# EUROPEAN PATENT SPECIFICATION

(11) **EP 0 504 278 B1**
(45) Date of publication and mention of the grant of the patent: **15.01.1997**
(21) Application number: 91901361.5
(22) Date of filing: 29.11.1990
(51) Int. Cl.: C12Q 1/68, C12Q 1/70, C12N 15/11, C12N 5/10

(54) **A NEW METHOD FOR DETECTING A SPECIFIC NUCLEIC ACID SEQUENCE IN A SAMPLE OF CELLS**
NEUES VERFAHREN ZUM NACHWEIS EINER SPEZIFISCHEN NUKLEINSÄURESEQUENZ IN EINER ZELLPROBE
NOUVELLE METHODE PERMETTANT DE DETECTER UNE SEQUENCE D'ACIDE NUCLEIQUE SPECIFIQUE A PARTIR D'UN PRELEVEMENT DE CELLULES

(30) Priority: 30.11.1989 US 443910; 06.04.1990 US 505833; 05.07.1990 US 548027
(43) Date of publication of application: 23.09.1992
(73) Proprietor: Pharmacia Biotech AB, 751 82 Uppsala (SE)
(72) Inventor: FROSTELL, Asa, S-75327 Uppsala (SE); NUNN, Michael, F., Solana Beach, CA 92075 (US)
(74) Representative: Widén, Björn
(86) International application number: US9006953
(87) International publication number: WO9108308

(56) References cited:
- EP-A- 0 393 744
- GB-A- 2 139 349
- US-A- 4 683 195
- US-A- 4 952 499
- ONCOGENE vol. 4, no. 9, September 1989, pages 1149 - 1151 I.K.HEWLETT ET AL.
- BLOOD vol. 72, no. 4, October 1988, NEW YORK,USA pages 1117 - 1123 S.KWOK ET AL.
- T. MANIATIS et al., "Molecular Cloning, A Laboratory Manual" published 1982 by MacGraw-Hill (N.Y.), see page 89.

## Description

### Technical Field

The present invention relates to a method for releasing genomic DNA from cells as well as for use of the method for detecting a specific nucleic acid sequence contained in a genomic DNA-containing aqueous cell sample.

### Background

Several methods for releasing DNA from cells for subsequent amplification via the polymerase chain reaction (PCR) have been reported. Typically, the cellular DNA is first purified from the cells by treatment with a detergent to solubilize cell components and a proteolytic enzyme to digest proteins that would otherwise remain bound to the DNA. This step is usually followed by extraction of the DNA with organic solvents. Finally, the extracted DNA is separated from residual organic solvents by steps such as ethanol precipitation of nucleic acids.

Recently, PCR has been performed on tissue culture cells whose DNA was released by lysing the cells at 95 degrees C in water. Saiki, et al., Nature, 324: 163-166 (1986). However, this method is inefficient because most of the cellular DNA remains in insoluble form. PCR has also been used to amplify DNA left in the supernatant from whole blood heated with alkali and centrifuged. Kogan et al., N. Engl. J. Med., 317: 785-990 (1987). The difficulty with this method is that the amount of supernatant liquid that can be added to a PCR reaction mixture, and thus the amount of target DNA, is limited by inhibitors in the supernatant liquid. This problem is particularly critical when the target for DNA amplification is present only in a minor fraction of cells in the sample, as in the case for infectious agents such as HIV [Ou et al., Science, 239: 295-297 (1988)] or for transgenes in recipient organisms.

EP-A-393 744 (filed before the filing date of the present application but published after that date) discloses a method for the extraction of nucleic acid from cells comprising the steps of cell isolation by density gradient centrifugation and cell lysis by heating at a temperature of between 95 and 120°C for between 2 and 15 minutes. The method may be applied to the detection of human retroviral pathogens, such as HIV-1 and HTLV-I.

### Brief Summary of the Invention

It has now been discovered that DNA can be released from cells in a manner that permits direct analysis for the presence of a specific nucleic acid sequence by the polymerase chain reaction.

According to the present invention genomic DNA is released from cells, such as lymphocytes, by first isolating the cells in an aqueous medium containing less than 80 mg/ml extracellular protein by using antibodies attached to a solid support which bind to the genomic DNA containing cells. The cells are then subjected to a temperature of at least about 105 degrees C for at least about 5 minutes.

The released genomic DNA can subsequently be assayed for the presence of a specific nucleic acid sequence, preferably by the polymerase chain reaction.

Assaying by the polymerase chain reaction comprises separating separating the strands of the double-stranded genomic nucleic acid, treating the separated single strands with a primer extension reaction mixture containing two primers, dNTPs and polymerase enzyme, under conditions such that the primers hybridize to the specific sequence and an extension product of each primer is synthesized by the enzyme. The primer extension products thus formed are separated into single strands which are again treated with the above primer extension admixture under similar conditions for primer hybridization and extension to form a primer extension product. The presence of the amplification product indicates the presence of the specific nucleic acid sequence. In preferred embodiments, detection of the PCR product is accomplished by treating, under hybridizing conditions, the product with an affinity-labeled polynucleotide and a lanthanide-labeled polynucleotide, each of labeled polynucleotides being capable of hybridizing to different regions of a single-stranded DNA of the PCR product to form a DNA duplex having affinity- and lanthanide-labels linked thereto.

The labeled DNA duplex thus formed is treated, under binding conditions, with a solid-phase affinity sorbent capable of binding the affinity-label of the DNA duplex to form a solid-phase lanthanide-labeled DNA duplex. The amount of the solid-phase lanthanide-labeled DNA duplex formed is then determined.

### Brief Description of the Drawings

In the drawings forming a portion of this disclosure:

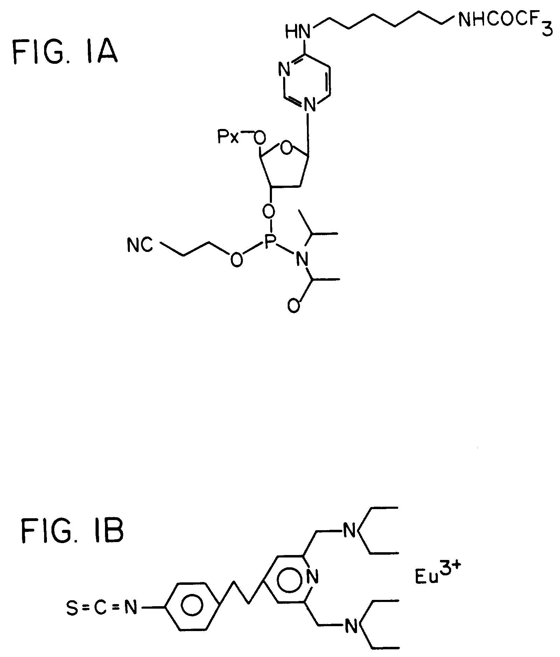

Figure 1, Panel A illustrates the modified cytidine residue used in the labeling of polynucleotide probe S9.

Panel B illustrates the europium chelate (W2014) used to label polynucleotide S9. One nanomole of modified cytidine was dissolved in 300 microliters (ul) of 0.5 M carbonate buffer (pH 9.8). Ten to twenty equivalents of the isothiocyanate of chelate W2014 per each amino group were added to the mixture and the pH adjusted to about 10.0. After reaction for about 16 hours, the labeled S9 probe was isolated by column chromatography.

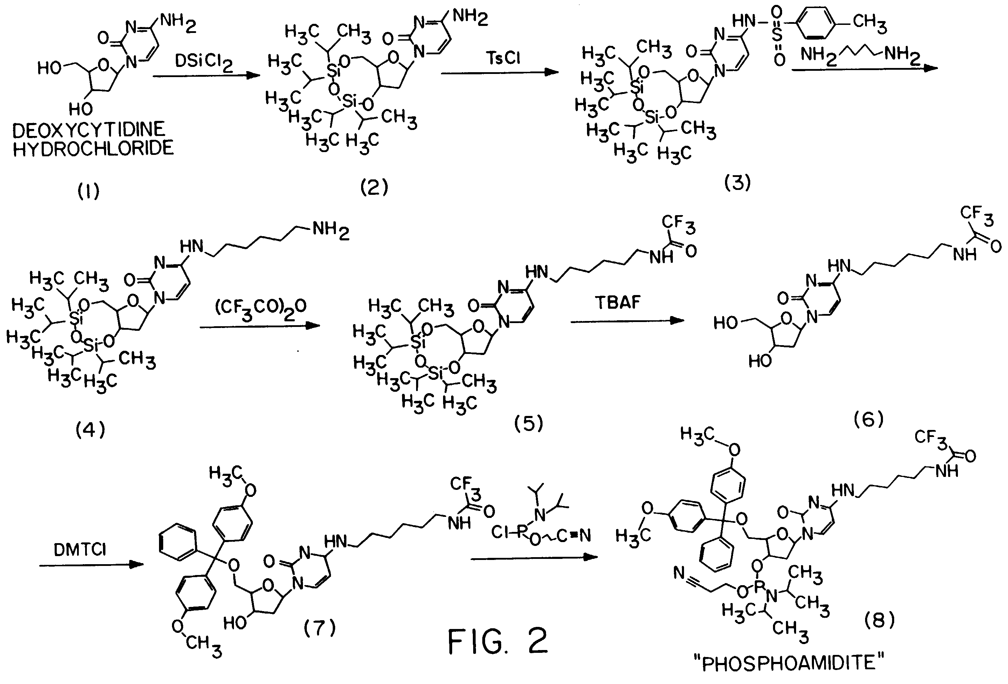

Figure 2 illustrates the scheme for the synthesis of the NH₂-modified deoxycytidine phosphoramidite.

### Detailed Description of the Invention

### A. Definitions

Nucleotide: a monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. When the nucleoside contains a phosphate group bonded to the 3' or 5' position of the pentose it is referred to as a nucleotide.
Base Pair (bp): a partnership of adenine (A) with thymine (T), or of cytosine (C) with guanine (G) in a double stranded DNA molecule.
Nucleic Acid: a polymer of nucleotides.
Gene: a nucleic acid whose nucleotide sequence codes for a RNA or polypeptide. A gene can be either RNA or DNA.
Complementary Bases: nucleotides that normally pair up when DNA or RNA adopts a double stranded configuration.
Complementary Nucleotide Sequence: a sequence of nucleotides in a single-stranded molecule of DNA or RNA that is sufficiently complementary to that on another single strand to specifically hybridize to it with consequent hydrogen bonding.
Conserved: a nucleotide sequence is conserved with respect to a preselected (reference) sequence if it non-randomly hybridizes to an exact complement of the preselected sequence.
Hybridization: the pairing of substantially complementary nucleotide sequences (strands of nucleic acid) to form a duplex or heteroduplex by the establishment of hydrogen bonds between complementary base pairs. It is a specific, i.e., non-random, interaction between two complementary polynucleotides that can be competitively inhibited.
Nucleotide Analog: a purine or pyrimidine nucleotide that differs structurally from a normal nucleotide, but is sufficiently similar to substitute for the normal nucleotide in a nucleic acid molecule.

### B. Methods For Releasing Cellular Nucleic Acids

As mentioned above, the method of the invention generally combines the following elements:
i) Isolating, in an aqueous medium, target cells, such as genomic DNA-containing cells, from extracellular protein, such as albumin, immunoglobulin, fibrinogen and the like by means of antibodies attached to a solid support which bind to the genomic DNA containing cells.
ii) Subjecting the isolated cells to a temperature of at least about 105 degrees centigrade (C) for a time period of at least about 5 minutes (min).

The nucleic acid so released can be used in a wide variety of molecular biological techniques requiring nucleic acid hybridization. For instance, control samples or standards for nucleic acid hybridization-based diagnostic assays, including those using Northern blot, Southern blot, primer extension, polymerase chain reaction (PCR) and like techniques, can be produced using the methods described herein. The methods of the present invention for releasing nucleic acid from cells can also be used to prepare samples for diagnostic assay by nucleic acid hybridization. For instance, nucleic acid released from cells can be assayed for the presence a pathogen-specific nucleic acid.

A "pathogen-specific" nucleic acid is a sequence of nucleotides forming a portion of the genome of a pathogen, such as a virus, bacterium, eucaryotic parasite, and the like. Exemplary viruses include human T-cell lymphotrophic (HTLV) virus, including Type I, Type II, or human immunodeficiency virus (HIV), such as HIV-1 and HIV-2, polio virus, adenovirus, parainfluenza virus, measles, mumps, respiratory syncytial virus, influenza virus, equine encephalomyelitis virus, hog cholera virus, Newcastle disease virus, fowl pox virus, rabies virus, feline and canine distemper viruses, feline leukemia virus, human cytomegalovirus, hepatitis Band C viruses, and the like. Bacterial pathogens include, B. pertussis, S. typhosa, S. paratyphoid A and B, C. diptheriae, C. tetani, C. botulinum, C. perfringens, B. anthracis, Y₆, pestis, P. multocida, V. cholerae, N. meningitides, N. gonorrhea, H. influenzae, T. pallidum and the like. Other Particularly important bacterial pathogens are those causing opportunistic infection in immunocompromised patients, such as E. coli, Klebsiella species, Pseudomonas species and the like.

Methods for determining the nucleotide sequence of a portion of a particular parasite's genome are well known in the art. In fact, useful portions of the nucleotide sequence of the genome of many pathogens have been reported. Group and/or type-specific nucleotide sequences can be used, depending, as is well known in the art, on the level of assay specificity desired.

### 1. Isolating Cells

By "extracellular protein" is meant protein molecules present as cell-free solutes in the blood. Extracellular proteins include immunoglobulins, albumin, fibrinogen, transferin, lipoproteins, alpha₂-macroglobulin, haptoglobin and the like.

Typically, the cells to be isolated are those present in the blood or a subpopulation thereof. Preferably, the isolated cells are lymphocytes, including T cells, B cells, cells of the monocyte/macrophage lineage, and the like. A particularly important subpopulation of circulating cells is defined by the CD4 receptor on the cell surface, i.e., CD4 positive cells.

The cells are "isolated" when the aqueous medium with which they have contact contains less than about 80 mg/ml, and preferably less than about 40 mg/ml, extracellular protein. Methods for determining the concentration of protein in an aqueous solution are well known. See, for example, Lowry et al., J. Biol. Chem., 193:265-75 (1951).

In preferred embodiments, the isolated cells are substantially free of red blood cells and/or cell free hemoglobin, including porphyrin compounds derived from heme under physiologic conditions. By "substantially free" is meant that the amount of hematin present will not inhibit the PCR reaction by more than about 10%, preferably no more than about 5% compared to a PCR reaction on an equivalent sample of DNA that has been purified by a standard phenol/ ethanol extraction procedure. It is preferred that the isolated cells be sufficiently free of red blood cells and/or cell free hemoglobin such that, upon lysis of all cells present in the isolated cell sample, the hematin concentration of the resulting solution will be less than 10 micromolar (uM), preferably less than 5 uM, more preferably less than 1.0 uM and most preferably less than 0.8 uM.

Lymphocytes can be isolated from blood by affinity chromatography using an antibody-based receptor that recognizes a lymphocyte-specific cell surface ligand or a ligand that is recognized by a lymphocyte-specific cell surface receptor. For instance, antibody molecules against the CD4 receptor, which is expressed by a subpopulation of lymphocytes, can be linked to a solid matrix, and then admixed with whole blood or a lymphocyte-containing portion thereof. The solid/liquid phase binding reaction admixture thus formed is maintained for a time period, typically predetermined, sufficient for the solid matrix-linked antibody molecules to bind any CD4 positive lymphocytes present in the sample to form a solid-phase complex. The solid-phase complex is separated from the other components of the binding reaction admixture, including extracellular protein, typically by washing with an aqueous buffer that does not lyse the cells, preferably an isotonic buffer. The cells thus isolated can be heat-treated as described herein, with or without separation from the antibody molecules to which they were bound.

Antibodies useful for isolating CD4 positive lymphocytes include those described in U.S. Patent No. 4,381,295 to Kung and Goldstein.

HIV infected lymphocytes can be isolated using CD4 as a receptor to bind HIV gp160 envelope precursor protein or HIV gp120 envelope protein expressed on the surface of HIV infected cells. The CD4 receptor is used as described above to bind HIV infected cells to a solid matrix which can then be separated from the remaining portion of the sample, including red blood cells, hemoglobin released from lysed red blood cells (cell free hemoglobin) and extracellular protein. Methods for producing and using CD4 receptor are described in International Patent Application No. PCT/US88/03592 (Publication No. WO 89/038143).

CD4 positive lymphocytes can be isolated in like manner using a ligand capable of being bound by CD4 linked to a solid matrix. Useful CD4-binding ligands include the HIV gp160 envelope precursor protein and the HIV gp120 envelope protein.

Useful solid matrices are well known in the art. Such materials include the cross-linked dextran available under the trademark SEPHADEX from Pharmacia Fine Chemicals, Piscataway, N.J.; agarose; beads of polystyrene abut 1 micron to about 5 millimeters in diameter available from Abbott Laboratories of North Chicago, IL; polyvinylchloride, polystyrene, cross-linked polyacrylamide, nitrocellulose or nylon-based webs such as sheets, strips or paddles; ore tubes or plates or the wells of a microliter plate such as those made from polystyrene, polypropylene, polyvinylchloride or polycarbonate.

Preferred matrices include paramagnetic and magnetic (magnetized or capable of being magnetized or subject to the influence of a magnetic field) particles or beads, such as iron-containing particles coated with (embedded in) a plastic or resin, preferably polystyrene, polyvinylchloride, polycarbonate or polypropylene. Exemplary magnetic beads are those available under the tradename DYNABEADS from Dynal, which are antibody coated superparamagnetic polystyrene beads 4.5 microns in diameter. The magnetic property of magnetic particles can be used to retain the particles during the process of separating the cells bound thereto from extracellular protein.

### 2. Heat Treatment of Cells

Isolated cells are heated to a temperature of at least about 105 C, preferably at least about 110 C and more preferably at least about 115 C. Usually, a temperature of no more than about 125 degrees C is needed.

The cells are exposed to the temperature for at least about 5 min, preferably at least about 10 min. and more preferably at least about 15 min. Usually, no more than about 25-30 min is needed.

It is desirable that the heating occurs under super-atmospheric steam pressure, typically pressure of about 10 to about 20 atmospheres, more typically a pressure of about 13 to about 17 atmospheres, usually about 15 atmospheres.

Typically, the heat treatment is accomplished by autoclaving the isolated cells for a time period at a temperature and super-atmospheric steam pressure sufficient to sterilize the sample. In preferred embodiments, the heat treatment is performed in the presence of nucleic acid primers so that the released nucleic acid, which is denatured into single strands during the heat treatment, will hybridize to the primers upon cooling.

### 3. Amplification Methods Preparation Of Polynucleotide Primers

The term "polynucleotide" as used herein in reference to primers, probes and nucleic acid fragments or segments to be synthesized by primer extension is defined as a molecule comprised of two or more deoxyribonucleotides or ribonucleotides, preferably more than 3. Its exact size will depend on many factors, which in turn depends on the ultimate conditions of use.

The term "primer" as used herein refers to a polynucleotide whether purified from a nucleic acid restriction digest or produced synthetically, which is capable of acting as a point of initiation of synthesis when placed under conditions in which synthesis of a primer extension product which is complimentary to a nucleic acid strand is induced, i.e., in the presence of nucleotides and an agent for polymerization such as DNA polymerase, reverse transcriptase and the like, and at a suitable temperature and pH. The primer is preferably single stranded for maximum efficiency, but may alternatively be double stranded. If double stranded, the primer is first treated to separate its strands before being used to prepare extension products. Preferably, the primer is a polydeoxyribonucleotide. The primer must be sufficiently long to prime the synthesis of extension products in the presence of the agents for polymerization. The exact lengths of the primers will depend on may factors, including temperature and the source of primer. For example, depending on the complexity of the target sequence, a polynucleotide primer typically contains 15 to 25 or more nucleotides, although it can contain fewer nucleotides. Short primer molecules generally require cooler temperatures to form sufficiently stable hybrid complexes with template.

The primers used herein are selected to be "substantially" complementary to the different strands of each specific sequence to be detected by synthesis or amplification. This means that the primer must be sufficiently complementary to nonrandomly hybridize with its respective template (target) strand. Therefore, the primer sequence may not reflect the exact sequence of the template. For example, a non-complementary nucleotide fragment can be attached to the 5' end of the primer, with the remainder of the primer sequence being substantially complementary to the strand. Such noncomplementary fragments typically code for an endonuclease restriction site. Alternatively, noncomplementary bases or longer sequences can be interspersed into the primer, provided the primer sequence has sufficient complementarily with the sequence of the strand to be synthesized or amplified to non-randomly hybridize therewith and thereby form an extension product under polynucleotide synthesizing conditions.

The polynucleotide primers can be prepared using any suitable method, such as, for example, the phosphotriester on phosphodiester methods see Narang et al., Meth. Enzymol., 68:90, (1979); U.S. Patent No. 4,356,270; and Brown et al., Meth. Enzymol., 68:109, (1979).

The selection of a primer's nucleotide sequence depends on factors such as its hybridization site on the nucleic acid relative to any second primer to be used, and the like. While several primer sequences may be selected and found to operate essentially equally well in ultimately detecting the target sequence, it is wise, in most applications, to choose these sequences from conserved regions of the target. Furthermore, the primer sequences should be analyzed for the possible formation of hair-pin loops which may impede their proper functioning, as well as for complementary sequences which may cause primer-primer hybrids, again impeding an effective use in the desired primer extension reaction. Generally, however, given a specific target target sequence to be detected, anyone skilled in the art may be expected to be able to design a multitude of sets of primer pairs, several of which are most likely to function with essentially equal efficiency in the primer extension reaction. Therefore, the sequences given in the present application should not be interpreted as limiting the scope of the invention but merely as examples of primer pairs which have been found to fulfill the favorable criteria given above, for a successful primer extension reaction.

If the sequence to be detected is in the form of double stranded genomic DNA, it is first denatured, typically by melting, into single strands. The DNA is subjected to a first primer extension reaction by treating (contacting) the DNA with a first polynucleotide synthesis primer having a preselected nucleotide sequence. The first primer is capable of initiating the first primer extension reaction by hybridizing to a specific nucleotide sequence, preferably at least about 10 nucleotides in length and more preferably at least about 20 nucleotides in length. This is accomplished by mixing the first primer, preferably a predetermined amount thereof, with the nucleic acids of the genomic DNA-containing sample, preferably a predetermined amount thereof, to form a first primer extension reaction admixture. The admixture is maintained under polynucleotide synthesizing conditions for a time period, which is typically predetermined, sufficient for the formation of a first primer extension reaction product. The product is then subjected to a second primer extension reaction by treating it with a second polynucleotide synthesis primer having a preselected nucleotide sequence. The second primer is capable of initiating the second reaction by hybridizing to a nucleotide sequence, preferably at least about 10 nucleotides in length and more preferably at least about 20 nucleotides in length, found in the first product. This is accomplished by mixing the second primer, preferably a predetermined amount thereof, with the first product, preferably a predetermined amount thereof, to form a second primer extension reaction admixture. The admixture is maintained under polynucleotide synthesizing conditions for a time period, which is typically predetermined, sufficient for the formation of a second primer extension reaction product.

In another strategy, the object is to detect a polynucleotide complement of the desired specific nucleic acid sequence such as the anti-sense strand of genomic dsDNA or the polynucleotide produced by subjecting mRNA to a reverse transcriptase reaction. Methods for producing such complements are well known in the art. The complement is subjected to the above-described second primer extension reaction.

The primer extension reaction is performed using any suitable method. Generally it occurs in a buffered aqueous solution, preferably at a pH of 6-9, more preferably at 6-8.5 and most preferably about 8. Preferably, a molar excess (for genomic nucleic acid, usually about 10⁶:1 primer:template) of the primer is admixed to the buffer containing the template strand. A large molar excess is preferred to improve the efficiency of the process. For polynucleotide primers of about 20 to 25 nucleotides in length, a typical ratio is in the range of 50 ng to 1 ug, preferably 250 ng, of each primer per 100 ng to 6 ug of genomic DNA.

The deoxyribonucleotide triphosphates (dNTPs) dATP, dCTP, dGTP, and dTTP are also admixed to the synthesis reaction admixture in adequate amounts and the resulting solution is heated to about 90C - 100C for about 1 to 10 minutes, preferably from 1 to 4 minutes. After this heating period the solution is allowed to cool to room temperature, which is preferable for primer hybridization. To the cooled mixture is added an appropriate agent for inducing or catalyzing the primer extension reaction, and the reaction is allowed to occur under conditions known in the art. The synthesis reaction may occur at from room temperature up to a temperature above which the inducing agent no longer functions efficiently. Thus, for example, if DNA polymerase is used as inducing agent, the temperature is generally no greater than about 40C.

The inducing agent may be any compound or system which will function to accomplish the synthesis of primer extension products, including enzymes. Suitable enzymes for this purpose include, for example, E. coli DNA polymerase I, Klenow fragment of E. coli DNA polymerase I, T4 DNA polymerase, other available DNA polymerases, reverse transcriptase, and other enzymes, including heat-stable enzymes, which will facilitate combination of the nucleotides in the proper manner to form the primer extension products which are complementary to each nucleic acid strand. Generally, the synthesis will be initiated at the 3' end of each primer and proceed in the 5' direction along the template strand, until synthesis terminates, producing molecules of different lengths. There may be inducing agents, however, which initiate synthesis at the 5' end and proceed in the 3' direction, using the same process as described above.

The newly synthesized strand and its complementary nucleic acid strand form a double-stranded molecule which they can form, each act as a marker for the specific nucleic acid sequence sought to be detected.

In preferred strategies, the first and second primer extension reactions are the first and second primer extension reactions in a polymerase chain reaction (PCR).

PCR is carried out by simultaneously cycling, i.e., performing in one admixture, the above described first and second primer extension reactions, each cycle comprising polynucleotide synthesis followed by denaturation of the double stranded polynucleotides formed. Methods and systems for amplifying a specific nucleic acid sequence are described in U.S. Patents No. 4,683,195 and No. 4,683,202, both to Mullis et al. and No. 4,889,818 to Gelfand et al.

Thus a method of detecting a specific nucleic acid sequence present in a sample of isolated, heat treated cells containing a double-stranded nucleic acid molecule may comprise the steps of:
(a) Separating the strands of the double-stranded nucleic acid molecule to form single-stranded templates.
(b) Treating the single-stranded templates with primer extension reaction mixture that contains (i) two polynucleotide primers that are selected so as to be sufficiently complementary to different strands of the specific sequence to hybridize therewith such that an extension product synthesized from one primer, when it is separated from its complementary nucleic acid strand, can serve as a template for synthesis of the extension product of the other primer, and (ii) an enzyme capable of producing primer extension products from said single-stranded templates, wherein said treatment is conducted under conditions such that the primers hybridize to the specific sequences and an extension product of each primer is synthesized by the action of the enzyme, said product being complementary to each single-stranded template.
(c) Separating the primer extension products formed in step (b) from the templates on which they are synthesized to produce single-stranded molecules.
(d) Treating, such as by admixing and maintaining, the single-stranded molecules produced in step (c) with the primer extension admixture of step (b) under hybridizing conditions, and for a predetermined time period, such that a primer extension product is synthesized using the single-stranded molecules formed in step (c) as a template.
(e) Detecting the presence of any primer extension product formed in step (d) and thereby the presence of a specific nucleic acid sequence in the isolated cell.

Detection of amplified nucleic acid product can be accomplished by any of a variety of well known techniques. In a preferred embodiment, the amplified product is separated on the basis of molecular weight by gel electrophoresis, and the separated products are then visualized by the use of nucleic acid specific stains which allow one to observe the discreet species of resolved amplified product present in the gel. The presence of a particular amplified product, that is one having a specific molecular weight when resolved by gel electrophoresis, indicates that the polynucleotides were present in the DNA duplex and acted as primers in the PCR reaction to produce multiple copies of a nucleic acid having a discreet nucleotide sequence that includes the target nucleotide sequence. Although numerous nucleic acid specific stains exist and would be suitable to visualize the electrophoretically separated nucleic acids, silver stain, or ethidium bromide stain are preferred.

Alternative methods suitable to detect the amplified nucleic acid include a range of well known methods. Thus, methods using radiolabelled are well established, see eg. Hames B.D. , and Higgins S.J. (editors) (1985). Nucleic acid hybridization. A Practical Approach IRL Press Oxford, Washington DC. Attaching enzymes to probes (see eg. Jablonski et al. Nucleic Acid Research (1986) 14:6115-6128, or flourescent labels Agrawal et al. (1986) Nucleic Acid Research 14:6227-6245, or utilizing time-resolved fluorometry, see, e.g., Syvänen et al. Nucleic Acid Research (1986), 14:5037-5048, or Oser et al. Nucleic Acid Research 16:1181-1196) constitute well known approaches to detect nucleic acid via hybridization and may be preferentially applied towards samples containing amplified target DNA. Furthermore, chemiluminescent assays involving protection of acridinium ester in labeled probes hybridized to target DNA (Gen Probe Inc. Technical Bulletin (1989)) constitute yet another means of utilizing labeled nucleic to detect specifically, complementery nucleic acid. These various methods indicate that given the presence of sufficent target nucleic acid, it is well established in the art that the presence of target nucleic acid may be demonstrated by a wide range of any one of effective labels attached to the probing DNA i.e., a polynucleotide probe or probe.

A labeled nucleotide residue present in a DNA renders the DNA itself labeled and therefore distinguishable over other nucleic acids present in a sample to be assayed. Detecting the presence of the label in the DNA and thereby the presence of the specific DNA sequence, typically involves separating the DNA from any labeled dNTP that is not present as part of a PCR product or duplex containing a PCR product.

In one preferred embodiment, an affinity based collection (ABC) assay method is used to detect the polynucleotide PCR product (see, e.a., Syvänen et al. (1986) Nucleic Acid Research, 14:5037-5048). In the method, a pair of probes is hybridized to a DNA strand containing the target nucleic acid sequence or its complement present in the PCR product. One of the probes is linked to an affinity label by which the formed hybrids can be isolated on a solid phase. The second (signaling) probe of the pair is linked to a label (sometimes referred to herein as a reporter group on signal generating means) that will render a primer extension product detectable. Typically such labels include radioactive atoms, chemically modified nucleotide bases, a lanthanide, such as europium, and the like.

An affinity label is a molecular entity capable of specifically (non-randomly) binding to another molecular entity, i.e., a receptor, such as an antibody or a ligand, such as an antigen. A commonly used affinity label includes biotin, and methods for its attachment to, eg. DNA, are known (See Sheldon et al., (1986), Proc. Nat. Acad. Sci. USA, 83:9085-9089). Biotin binds strongly to streptavidin, or to avidin, but anti-biotin antibodies may also utlized as complementary binding agent. Another affinity label is dinitrophenol against which antibodies are easiliy made and can be used as the second component in an affinity label pair. Other small molecules, such as digoxin (again, anti-digoxin antibodies may be used as second component) are equally feasible in such systems. Thus, any such system provides a means of isolating the DNA following the application of the present invention to release the target nucleic acid.

In the ABC assay method the affinity-labeled oligonucleotide hybridizes to the specific nucleic acid of interest to form a complex (DNA duplex) containing the affinity label. The affinity-labeled DNA duplex thus formed is then bound to an affinity sorbent comprising an agent that specifically binds the affinity label, which agent is operatively linked to a solid (aqueous insoluble) support. Useful solid matrices are well known in the art. Such materials include cross-linked dextran available under the trademark SEPHADEX from Pharmacia Fine Chemicals (Piscataway, N.J.); agarose; polystyrene beads about 1 micron to about 5mm in diameter available from Abbott Laboratories of North Chicago, IL; polyvinyl chloride, polystyrene, cross-linked polyacrylamide, nitrocellulose or nylon-based webs such as sheets, strips or paddles, or tubes, plates or the wells of a microtiter plate such as those made from polystyrene or polyvinylchloride.

Hybridizing of the signaling probe to a DNA strand containing the specific nucleic acid sequence to be detected renders the DNA duplex thus formed detectable. When both the affinity-labeled probe and the signaling probe are hybridized to a DNA containing the specific nucleic sequence to be detected, a DNA duplex containing the signaling probe and the affinity label is formed.

The phrase "hybridizing conditions" and its grammatical equivalents, when used with a maintenance time period, indicates subjecting the hybridization reaction admixture, in the context of the concentrations of reactants and accompanying reagents in the admixture,- to time and temperature conditions sufficient to allow the polynucleotide to anneal with the target (specific) nucleic acid sequence and form a double stranded DNA duplex. Such time and temperature conditions required to accomplish hybridization depend, as is well known in the art, on the length of the polynucleotide segment to be hybridized, the stringency of hybridization desired, and the presence of salts or additional reagents in the hybridization reaction admixture as may affect the kinetics of hybridization. Methods for optimizing hybridization conditions for a given hybridization reaction admixture are well known in the art.

Typical hybridizing conditions include the use of solutions buffered to pH values between 5 and 9, and are carried out at temperatures from 18 to 70 degrees C, for time periods from 0.5 minutes to 24 hours.

Hybridization can be carried out in a homogeneous or heterogeneous format as is well known. The homogeneous hybridization reaction occurs entirely in solution, in which both the polynucleotide probe and the nucleic acid sequences to be hybridized (target) are present in soluble forms in solution. A heterogeneous reaction involves the use of a matrix that is insoluble in the reaction medium to which either the probe or target nucleic acid is bound.

The DNA duplex containing the affinity and signaling labels is bound to an affinity sorbent by admixing the duplex and the sorbent and maintaining the binding reaction admixture thus formed under binding conditions for a time period sufficient for the affinity label to bind the affinity sorbent. DNA duplex bound is then separated from non-bound materials, typically by washing.

"Binding conditions" are those combinations of parameters such as pH value, salt concentration, temperature and the like of the binding reaction admixture that promote specific (non-random) binding of the affinity label to the specific binding agent of the affinity sorbent. Such conditions depend on the particular combination affinity label and affinity sorbent used, as is well known in the art. Binding conditions are typically chosen to promote detectable binding within a predetermined time period of about 1 minute to 14 hours, preferably within about 5 minutes to about 90 minutes.

In preferred embodiments, the first and second polynucleotide synthesis primers are complementary to portions of a retroviral long terminal repeat genomic region, thereby permitting amplification of a plurality of DNA segments using a single pair of primers.

In another preferred embodiment, two pairs of probes are utilized, one pair for each individual strand of DNA produced in the PCR amplification step, thereby doubling the signal obtainable from a given PCR amplification product.

In any event, the affinity-labeled oligonucleotide probe and the signaling-labeled oligonucleotide probe have nucleic acid sequences complementary to sufficiently different regions of the DNA strand to be detected that they are both capable of hybridizing to said strand to form a DNA duplex labeled with both labels. The probe-hybridization regions can be defined by contiguous, adjacent or partially overlapping nucleotide sequences. Preferably, the probes hybridize to regions separated by about 1 to about 20 nucleotide bases.

Radioactive elements operatively linked to or present as part of a dNTP provide a useful labeling means to facilitate the detection of a DNA primer extension product. A typical radioactive element is one that produces beta ray emissions. Elements that emit beta rays, such as ³H, ¹⁴C, ³²P, and ³⁵S represent a class of beta ray emission-producing radioactive element labels. However, gamma-ray emitters such as the Iodine isotopes 125 or 131 may also be used for labeling oligonucleotides.

Alternatives to radioactively labeled dNTPs are dNTPs that are chemically modified to contain metal complexing agents, biotin-containing groups, fluorescent compounds, and the like.

One useful metal complexing agent is a lanthanide chelate formed by a lanthanide and an aromatic beta-diketone, the lanthanide being bound to the nucleic acid or polynucleotide via a chelate forming compound such as an EDTA-analogue so that a fluorescent lanthanide complex is formed. See U.S. Patents No. 4,374,120, and No. 4,569,790 and published International Patent Applications No. EP0139675 and No. W087/02708. A preferred lanthanide is a europium.

Biotin or acridine ester-labeled oligonucleotides and their use in polynucleotides have been described. See U.S. Patent No. 4,707,404, published International Patent Application EP0212951 and European Patent No. 0087636. Useful fluorescent marker compounds include fluorescein, rhodamine, Texas Red, and the like.

Another approach for detecting the presence of a specific nucleic acid sequence, the deoxyribonucleotide triphosphates (dNTPs) used in the primer extension reaction include a signaling label as described above.

Thus, a method based on the present invention for detecting a specific nucleic acid sequence contained in a blood sample, may comprise the steps of:
(a) isolating, in an aqueous medium, peripheral blood mononuclear (PBM) cells from said blood sample by antibody-based affinity chromatography, thereby forming an admixture containing extracellular protein at a concentration of less than 80 mg/ml.;
(b) heating said admixture to a temperature of about 105 degrees C to about 125 degrees C for a time period of at least about 10 minutes, thereby forming a heat treated sample;
(c) subjecting a portion of said heat treated sample to at least one cycle of a polymerase chain reaction using a first polynucleotide synthesis primer capable of initiating synthesis of a complement of said specific nucleic acid sequence and a second polynucleotide synthesis primer capable of initiating synthesis of said specific nucleic acid sequence;
(d) treating under hybridizing conditions, the polymerase chain reaction product with an affinity-labeled polynucleotide and a lanthanide-labeled polynucleotide, each of said polynucleotides being capable of hybridizing to different regions of a single-stranded DNA of said polymerase chain reaction product to form a DNA duplex having affinity- and lanthanide labels linked thereto;
(e) treating said DNA duplex, under binding conditions, with a solid-phase affinity sorbent capable of binding said affinity-label of said DNA duplex to form a solid-phase lanthanide-labeled DNA duplex; and
(f) determining the amount of said solid-phase lanthanide-labeled DNA duplex formed.

In preferred embodiments, the affinity label is biotin, the solid-phase affinity sorbent is streptavidin or avidin linked to an aqueous-insoluble solid matrix, and the lanthanide-label is Europium.

### Examples

The following examples are intended to illustrate, but not limit, the scope of the invention.

The following terms used in the examples are trademarks, some of them registered in at least some countries: Speed-Vac®, NAP™-10, PD™-10, Delfia®, Dynabeads®, Tween®, Plateshake™, Thermocycler™, Triton®, Arcus®, Ficoll®, Sephadex®.

### 1. Preparation of HIV-positive Control Cells

The cell line COS-10-11.1 contains a single intact copy of an HIV-1 genome which contains a mutation rendering the virus replication incompetent. This line serves as a model for an HIV infected cell and is the source for HIV DNA used in these studies.

The mutated HIV-1 DNA was generated from the pHXB2CG plasmid of the HTLV-IIIB strain of HIV-1, which contains a unique cleavage site for the methylation-sensitive restriction endonuclease Cla I located 41 base pairs downstream from the initiation codon for the viral core antigens. Genbank Data Library Supplement to the Sequence Analysis Software Package of the Genetics Computer Group, Version 6.0, February 1989, Accession No. KO3455, University of Wisconsin, Madison, Wisconsin. Non-methylated pHXB2CG was produced by transforming dam E. coli, amplifying the transformant and isolating plasmid DNA therefrom using standard techniques. 1.2 micrograms of isolated pHXB2CG were cleaved with Cla I, and the sequences surrounding the Cla I site were then removed by digestion with one unit of the endonuclease Bal31 (New England Biolabs) in 50 microliters of a buffer of 600 mM NaCl, 12 mM MgCl₂, 1 mM EDTA, 10 mM Tris pH 8.0 at 30 degrees C (30C). See, Maniatas et al., Molecular Cloning: A Laboratory Manual, Cold Spring Harbor Laboratory, Cold Spring Harbor N.Y. At 1 minute (min), 2 min, 5 min, 10 min, and 30 min 20% of the reaction was removed and the digestion stopped by extraction with a mixture of phenol and chloroform. After concentration by precipitation in ethanol the digested plasmid DNAs were ligated to form closed circular molecules using T4 DNA Ligase. After inactivation of the ligase the ligated molecules were retreated with Cla I in order to cut any molecules without deleted nucleotides and the DNAs were inserted into the JM83 strain of E. coli by the calcium chloride method described in Maniatas et al., supra. Individual colonies of transformed bacteria were grown and mutant plasmids isolated. Plasmid 10 contained a small deletion and was purified and further characterized by DNA sequencing. At least 112 base pairs and no more than about 115 base pairs were deleted upstream from nucleotide sequence position 847 in pHXB2CG removing the Cla I restriction site, the core antigen initiation codon and the splice donor signal for the generation of viral messenger RNAs from the HIV genome.

One microgram of the mutant 10 plasmid DNA was introduced into one million COS-7 cells (ATCC CRL 1651) together with the 10 nanograms pSV2neo plasmid carrying the resistance marker for the drug G418, and after several weeks of selection in 400 microgram/ml G418, resistant colonies of cells could be isolated. Several colonies were grown to large numbers and the cellular DNA extracted. Southern blot analysis indicated that COS clone 10-11 contained one intact copy of the HIV gag region. Metabolic labeling the proteins of this cell line with 35S-methionine and immunoprecipitation with AIDS patients sera indicated that it does not express any appreciable level of HIV antigens. The line was sucloned, and clone COS 10-11.1 was characterized and found to contain a single copy of the HIV target sequence per haploid genome by Southern analysis.

### 2. Synthesis of Primers for Targeting Proviral HIV-1 DNA

Two primers, S1 and S2, with the following sequences were prepared:

S1:  5' GGAACCCACTGCTTAAGCC  3'

S2 5' GGTCTGAGGGATCTCTAG 3'

Each primer was synthesized on the Gene Assembler (Pharmacia LKB Biotechnology) using phosphoramidite chemistry according to a standard protocol as supplied in the manual of the manufacturer of the instrument. The purification of each synthesized oligonucleotide was accomplished through preparative polyacrylamide gel electrophoresis as described below.

All oligonucleotides were synthesized on the 1.3 umole scale.

Deprotection was carried out as follows. After completion of the program the support was placed into an Eppendorf tube with the casette's wider end is up. The casette was centrifuged briefly in an Eppendorf centrifuge at less than 4,000 rpm, and then removed into a new tube. One ml of concentrated ammoniumhydroxide was added and the tube was placed into a boiling rack and keep at 55°C for 24 hours. Subsequently, the casette was removed from the ammoniumhydroxide solution into a new tube, and centrifuged again to recover all of the ammoniumhydroxide solution. The volume was adjusted to 2.5 ml with H₂O. Then, the ammoniumhydroxide was exchanged to H₂O by running the deprotected oligonucleotide over a PD-10 column (Pharmacia LKB Biotechnology) using H₂O as elution buffer. The oligonucleotide was then evaproated to dryness in a Speed-Vac (Savant).

Purification of oligonuclotides was carried out essentially as detailed in "Molecular Cloning - A Laboratory Manual (1982) Ed. Maniatis. T., Cold Spring Harbor Laboratories, Cold Spring Harbor.

A 10% urea polycrylamide gel (30% urea, 10% polyacrylamide) was prepared, and lx TBE as electrophoresis buffer, prerun at 340V for 30 minutes. The oligonucleotide was suspended in 60 ul of H₂O and mixed with 60 ul of the dye-mix (95% formamide, 10mM EDTA, 0.01% bromphenolblue, 0.01% Xylenolblue). Excess of urea was removed by displacing with 1xTBE. Sample was applied on the top of the gel (over the whole length of the gel) and the gel run at 340V until the lower dye band had migrated to the lower edge of the gel (about 1.5 hours). The gel was removed and placed on a TLC-sheet for UV-shadowing, using the longwave UV-lamp to locate the band of interest. The band was carefully removed using a razor blade. Subsequently, the oligonucleotide was eluted from the gel by using 1xTBE in the Epigel D-system (Epigene) into DEAE cellulose. After at least 2 hours with 100V in 1xTBE buffer, the oligonucleotide was eluted from the DEAE-cellulose precipitation; 10 mM MgCl₂ were added to insure good recovery. The oligonuclotide was resuspended in 1 ml of 20mM HEPES pH 7.5 and 100 uM EDTA, and run over two separate NAP-5 columns using the same buffer as eluent. The concentration of oligonucleotide was determined by measurement at 260nm in a UV-spectrophotometer using the HEPES-EDTA buffer as blank and using for calculation, that 1 O.D. corresponds to 33 ug/ml of oligonucleotide.

In Table 1, the oligonucleotides used as primers and probes for the detection of pathogen-derived nucleic acid used to illustrate the present invention are shown.

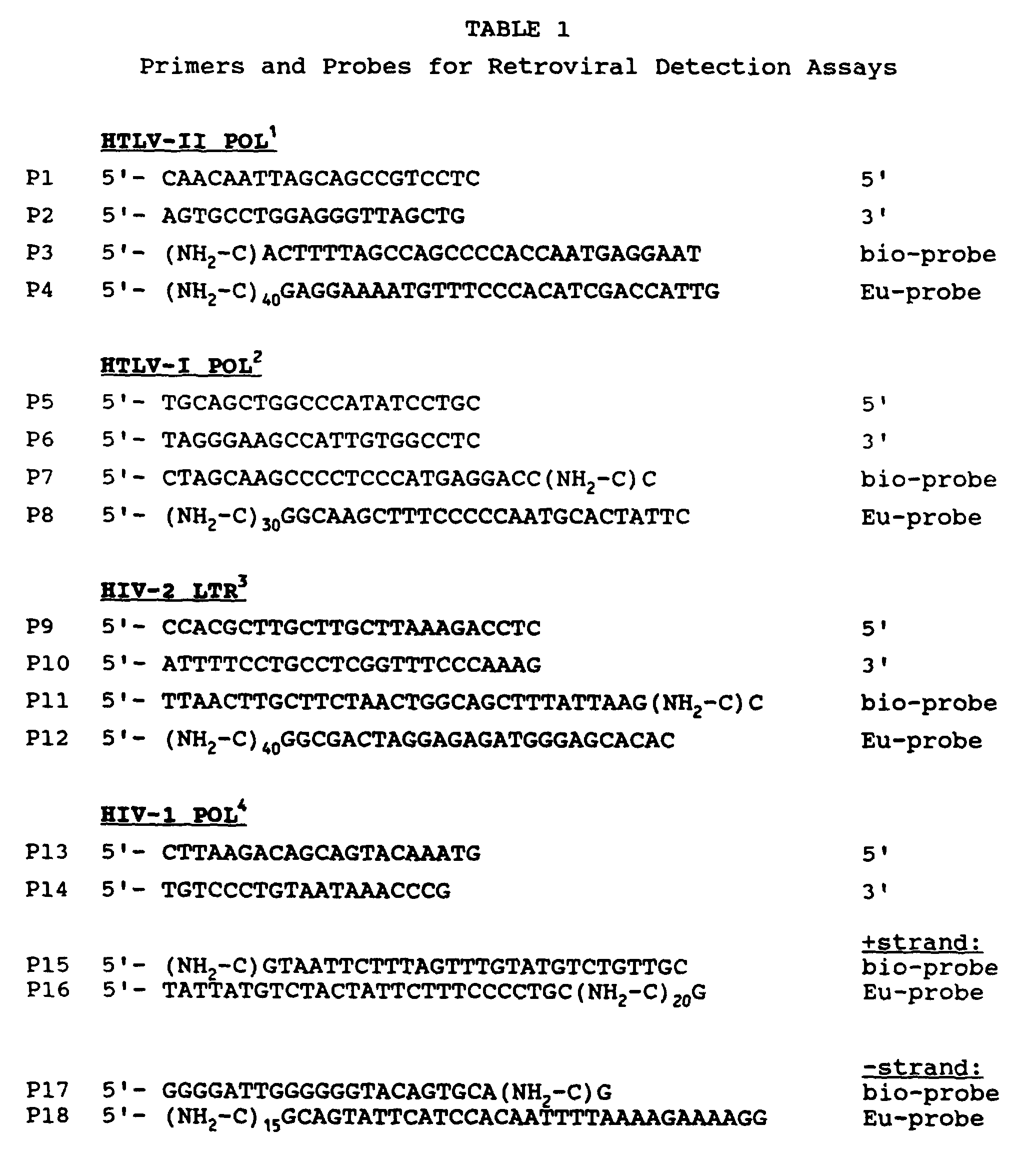

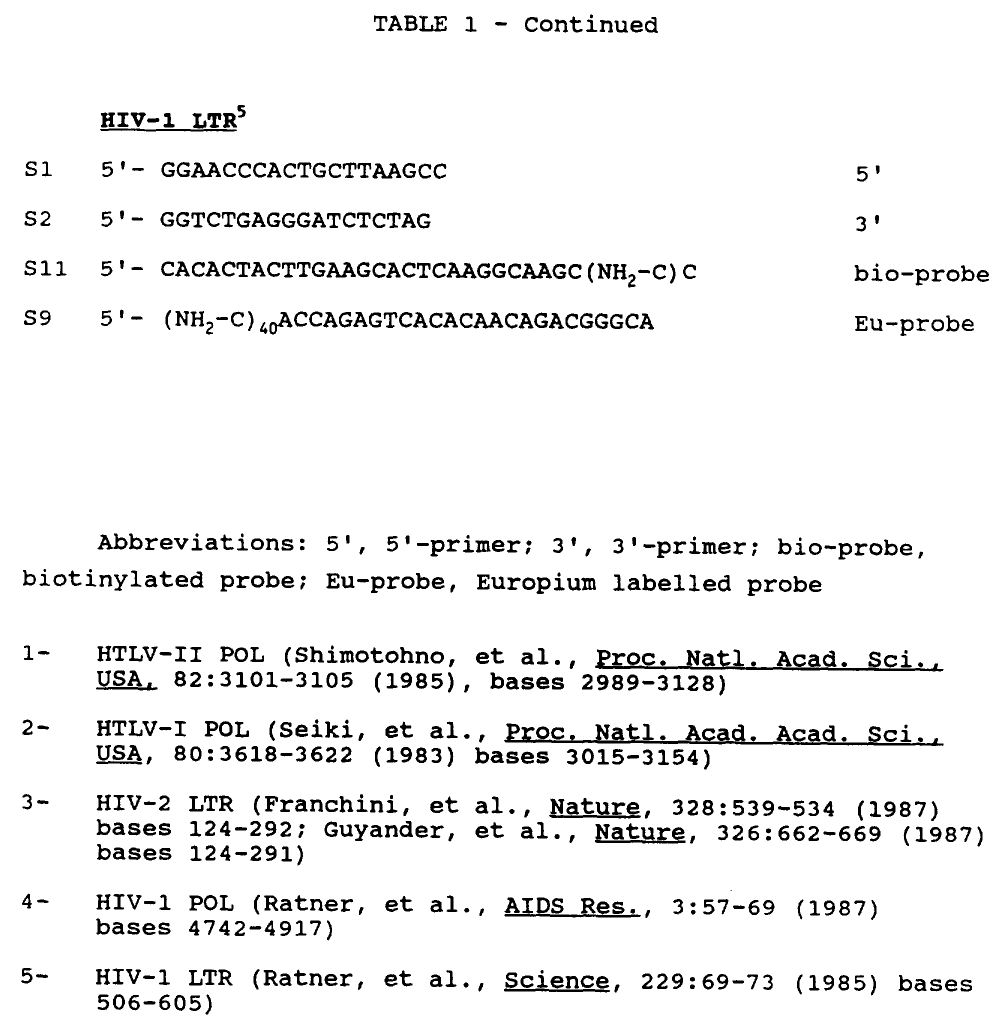

### 3. Labeling Polynucleotides with Europium

Polynucleotide S9 was labeled with europium by first synthesizing an amino modified deoxycytidine phosphoramidite (Figure 1A), synthesizing a polynucleotide containing the modified deoxycytidine and coupling the europium to the resulting polynucleotide.

### a. Synthesis of the Amino-Modified Deoxycytidine Phosphoramidite.

The overall scheme for the synthesis of the NH₂-modified deoxycytidine phosphoramidite is outlined in Figure 2. Pyridine, tetrahydofurane and acetonitrile were dried by reflux over calcium hydride for several hours and thereafter distilled. Triethylamine and diisopropylethylamine were refluxed over solid potassium hydroxide and distilled.

Tetrabutylammonium fluoride (TBAF x 3H₂O) was dried by coevaporation with dry acetonitrile to a final concentration of 0.5 M.

Tetraisopropyldisilocyldichoridate (TIPDSiCl₂) was synthesized according to Markiewicz, J. Chem.Pes., 241:173-181 (1979) and (2-cyanoethoxy) - N,N-diisopropylamino-chlorophospine according to Clausen et al., Recl. Trar. Chim. Pay-Bas, 104:119-122 (1985). All commercially available reagents were purchased from either Aldrich Chemical Co. (Milwaukee, WI) or Sigma (St. Louis, MO).

Thin layer chromatography (TLC) was carried out on precoated silica gel plates (Merck, 60 F254) using the following solvent systems: A, MeOH/CHCl₃ (10:90 v/v); B, MeOH/CHCl₃ (2080 v/v); C, n-propanol/NH₃ (aq)/H₂O (65:15:5 v/v); or D, n-hexane/ethyl acetate/triethylamine (60:30:10 v/v).

The short column chromatography separations were carried out using silica gel (40-63 µm, Merck G 60).

NMR spectra were recorded using a Jeol JNM 6X400 spectrometer, with either TMS as internal standard or 85% phosphoric acid as external standard.

### (i) 4-N-(p-Toluenesulphony)-3',5'-O-(1,1,3,3-tetraisopropyldisiloxane-1,3 -diyl)-deoxycytidine (3).

The title compound was prepared starting from deoxycytidine hydrochloride (1) (5.27 g,20 mmole) in dry pyridine (200 ml) and TIPDSiCl₂ (8.2 g, 26 mmole). After overnight stirring at room temperature (RT), the reaction was checked by TLC using systems. The TLC showed complete disappearance of (1) in system B and presence of a single product (2), R_{f}=0.21 in system A. Toluene-4-sulphonylchloride (6.1 g; 32 mmole) and dry diisopropylethylamine (5.5 ml, 32 mmole) were added. The reaction mixture was stirred at RT overnight, and the red colored mixture (3) was worked up by pouring it into saturated NaHCO₃ and extracting with CHCl₃ (3 x 200 ml). The organic phase was evaporated and coevaporated with toluene. The residue was then purified by short silica gel column chromatography (2% ethanol in CHCl₃ in the mobile phase).
Yield: 10.25 g (82%). R_{f}=0.60 in system A. ¹H NMR (CDCl₃): 7.84 (d, 2H, J=8.3 Hz); 7.81 (d, 1H, J=7.8Hz) H₅; 7.30 (d, 2H, J=8.3Hz); 5.98 (d, 1H, J=7.8Hz) H₆; 5.90 (t, 1H) H₁'; 4.50 to 3.70 (m, 4H) H₃'+ H₄' + H₅'; 2.60 to 2.20 (m, 2H) H₂'; 2.42 (s, 3H) tosyl; 1.20 to 0.90 (m, 28H).

### (ii) 4-N-(6-aminohexyl)-3',5'-O-(1,1,3,3,-tetraisopropyldisiloxane-1,3-diyl) -deoxycytidine (4).

Compound (3) (Markiewics et al., In the abstracts from the "7th International Round Table Nucleosides, Nucleotides and the Biological Applications," Konstanz (F.R.G.) at p.32, (1986)) (7.0 g, 14.9 mmole) described by Markiewicz et al., in the abstracts from 17th International Round Table Nucleosides, Nucleotides and the Biological Applications, p. 32, Konstanz (F.R.G.) (1986) and 1,6-diaminohexane (17.3 g, 149 mmole) were dissolved in dry pyridine (200 ml). The reaction mixture was stirred at 60°C for 18 hours. TLC showed a complete consumption of the starting material (3) and formation of product (4), R_{f}=0 in both systems A and B. The cooled reaction mixture was worked up by partitioning between saturated NaHCO₃ and CHCl₃. The combined organic phase was evaporated, coevaporated with toluene and purified by flash chromatography on silica gel, eluted first with 15% ethanol in CHCl₃ and then with 35% ethanol, 3% triethylamine in CHCl₃. Yield: 6.27 g (74%) - oil, R_{f}=0.53 in system C. ¹H NMR (CDCl₃): 7.75 (d, 1H) H₅; 6.10 (t, 1H) H₁'; 5.58 (d, 1H) H₆; 4.38 (q, 1H) H₃'; 4.15 to 3.70 (m, 3H) H₄' + H₅'; 3.43 (m, 2H); 2.70 (m, 2H); 2.55 to 2.25 (m, 2H) H₂'; 1.65 to 1.20 (m, 8H); 1.20 to 0.90 (m, 28H).

### (iii) 4-N-(6-Trifluoroacetamidohexyl)-5'-O(4,4-Dimethoxytrityl)-deoxycytidine (7).

The three step conversion of (4) to (7) was performed without isolation of intermediate products (5) and (6).

Compound (4) (6.0 g, 10.5 mmole) was coevaporated with dry pyridine, dissolved in dry pyridine (50 ml) and trifluoroacetic anhydride (3.3 g, 15.75 mmole) was added. The mixture was stirred at RT for 30 minutes and worked up by standard bicarbonate-chloroform extraction. The organic phase was evaporated, coevaporated with toluene and the residue dissolved in dry tetrahydrofurane (200 ml). To this solution TBAF 0.5 M in acetonitrile (50 ml, 25 mmole) was added and the mixture was stirred for 5 minutes at RT, while the complete conversion of (5) to (6) was followed using TLC, system A.

The reaction mixture was again evaporated to dryness, coevaporated twice with pyridine (2 x 100 ml) and dissolved in dry pyridine (100 ml).
Dimethoxytrityl chloride (4.75 g, 14 mmole) was added and the mixture was stirred at RT for 6 hours.

After standard work up, evaporation of the organic phase and coevaporation with toluene, the title compound (7) was isolated using silica gel column chromatography (3% ethanol in CHCl₃, in the mobile phase).
Yield: 5.11 g (67%) oil R_{f}=0.51 in system A. ¹H NMR (CDCl₃): 8.05 (d, 1H) NHCOF₃; 7.70 (d, 2H, J=7.5 Hz) H₅; 6.14 (t, 1H) H₁'; 5.70 (d, 1H, J=7.5 Hz) H₆; 4.66 (q, 1H) H₃'; 3.95 to 3.75 (m, 3H) H₄', H₅'; 3.37 (t, 2H); 3.29 (t, 2H); 2.50 to 2.23 (m, 2H) H₂'; 1.62 to 1.30 (m, 8H); 1.15 to 0.9 (m, 28H).

### iv) 4-N-(6-Trifluoroacetamidohexyl)-5'O-(4,4-dimethoxytrityl)-deoxycytidine-3, -(2-cyanoethyl)-N,N -diisopropylphosphoramidite (8).

Compound (7) (5.0 g, 6.9 mmole) was coevaporated twice with toluene, dissolved in dichloromethane (50 ml). To this mixture, cooled to 0°C, diisopropylethylamine (4.46 g, 34.5 mmole) followed by (2-cyanoethoxy)-N,N-diisopropylamino-chlorophosphine (3.25 g, 13.8 mmole) were added and the mixture was stirred at RT for 30 minutes. The reaction was followed by TLC in system D. Complete consumption of (8) was observed. The mixture was poured into a cooled saturated solution of sodium bicarbonate (200 ml) and extracted with CH₂Cl₂. The combined organic layer was evaporated, coevaporated with toluene and purified on short silica gel column chromatography (hexane/CH₂Cl₂/Et₃N, 30:50:20 v/v) giving the compound (8) as a colorless mass after precipitation from cold hexane (-70°C).
Yield: 5.5 g (86%), R_{f}=0.67 in system D. ³¹P NMR (CDCl₃): 149.41 and 149.46.

### b. Synthesis of modified oligonucleotides for with amino-substituted cytidine mononucleotide derivatives.

To attach the labeled europium chelate to the oligonucleotide, an oligonucleotide containing a stretch of amino-substituted cytidine mononucleotide derivatives (mod C Compound 8; See Figure 1A and Example 3a.) at the 5' end of the probe was used. The synthesis involving residues containing nucleotides modified for attachment of labels to the oligonucleotide was carried out analogous to the procedure used for the other B-cyanoethyl amidites and the cytidine derivative (Figure 1A) was placed into the free position available on the Gene Assembler. The purity of the isolated oligonucleotide was monitored by chromatography on an FPLC column( PEP RPC HR 5/5, Pharmacia LKB Biotechnology) using gardient elution (buffer A, 100mM TEAA, 10% MeCN; buffer B, 100mM TEAA, 30% MeCN (Gradient: 0-100% B in 50 minutes, with a flow rate of 1ml/min. The detection was achieved through monitoring at 260 nanometers.

### c. Labelling of oligonucleotide S9 with Europium

To attach the Eu³⁺-chelate to the oligonucleotide, a stretch of amino-substituted cytidine mononucleotide derivatives ("Modified C", "modC") at the 5' end of the probe was utilized.

The sequence of the oligonucleotide S9 is as follows: S9 5'(modC)₄₀ACCAGAGTCACACAACAGACGGGC 3' and was synthesized and purified as detailed in the examples given above.

The oligonucleotide S9 was suspended in 500 ul of distilled H₂O and the solution passed through a NAP-5 column (Pharmacia LKB Biotechnology), equilibrated with distilled water. After elution with distilled water and collection of the effluent (1 ml), it was passed through a NAP-10 column (Pharmacia LKB Biotechnology) using distilled water and the eluate (1.5 ml) collected.

The concentration of the oligonucleotide S9 was determined by measuring the optical density at 260 nm in a UV spectrophotometer calibrated with distilled water as blank and the concentration of oligonucleotide calculated as (ug/ml) = 33xA, where A is the recorded absorbance at 260 nm.

The oligonucleotide (10 nmol) was evaporated to dryness using a Savant Speed-Vac concentrator and resuspended in 50 ul distilled H₂O. The pH was adjusted to 9.5 by adding 1 M Na₂CO₃ (approximately 2.5 ul; final concentration of sodium carbonate=50 mM). Then, a 400 fold molar excess of Eu³⁺ chelate W2014 (see compound 13 in Scheme 3 described in European Patent applications 88850218.4, Pub. No. 0298939, filed June 6, 1988 by Kwiatkowski, et al., and also shown in Figure 1B) was added, and the reaction mixture kept at 4°C overnight.

The reaction mixture was subsequently applied to a column (50x1 cm) of DNA Grade Sephadex G-50 (Pharmacia LKB Biotechnology), equilibrated and eluted with 10 mM HEPES buffer pH 7.5, containing 50 mM EDTA. Fractions (20 x 1 ml) were collected and monitored at 260 nm. The Eu-profile was determined by mixing 1 ul of each fraction to 1 ml of Delfia Enhancement Solution (U.S. Patent No. 4,565,790, issued 1/21/1986), followed by measurement of the flourescence using an Arcus fluorometer (Pharmacia Wallac). The fractions containing the first major peak were pooled and the amount of recovered Europium modified oligonucleotide was determined by UV-spectrophotometry.

The Eu-labeled oligonucleotide S9 was diluted to a concentration of 5.6 ug/ml in 10 mM HEPES buffer, pH 7.5, containing 50 mM EDTA and stored in 0.5 ml aliquots at -20°C.

### 4. Biotinylation

### a. Biotinylation of Oligonucleotide S11:

To attach the biotin molecule to nucleic acid, a residue of the amino-substituted cytidine mononucleotide derivative ("Modified C") at the penultimate position of the 3' end of the DNA probe is utilized.

The sequence of the Modified C containing Oligonucleotide S11 is as follows:

S11 5'-CACACTACTTGAAGCACTCAAGGCAAGCmodCC-3'

The oligonucleotide S11 was synthesized on the Gene Assembler using phosphoramidite chemistry according to a standard protocol.

The oligonucleotide S11 was suspended in 500 ul of distilled H₂O and passed through a NAP-5 column equilibrated with distilled water. Subsequently, the column was eluted with distilled water and the effluent (1 ml) collected. The collected effluent was then passed through a NAP-10 column using distilled water in an analogous fashion and the concentration of the effluent determined be measuring the optical density at 260 nm in a UV spectro-photometer calibrated with distilled water as blank. The concentration of the oligonucleotide was calculated by using the relationship ug/ml+ 33xAbsorbance at 260 nanometers.

The sample was then transferred to Eppendorf tubes and taken to to dryness using a Savant Speed-Vac concentrator. After resuspension in 50 ul distilled H₂O the pH was adjusted to 9.5 by adding 1 M Na₂CO₃ (approximately 2.5 ul; final concentration of sodium carbonate=50 mM). An appropriate amount (2-5mg) of Biotin-amidocaproate N-hydroxysuccinimide ester (Sigma, Molecular weight = 454) was taken up in 100 ul dry N,N-dimethylformamide (DMF). A 40-fold molar excess of the DMF solution of the active biotin-amidocaproate N-Hydroxysuccinimide ester was mixed with the Modified C oligonucleotide S11 solution and allowed to stand at room temperature for 3 hours.

The reaction product was diluted to 500 ul with distilled H₂O and passed through a NAP-5 column, equilibrated with 10mM HEPES buffer, pH 7.5 containing 50 uM EDTA. The column was eluted with the same buffer, the effluent (1 ml) was collected and again passed through a column (NAP-10, Pharmacia LKB Biotechnology), using 10 mM HEPES buffer, pH 7.5, containing 50 uM EDTA in an analogous fashion. The volume of the final effluent constituted 1.5 ml.

### b. Biotinylation of BSA

Ten mg of bovine serum albumin (BSA,Sigma A-7906, Molecular weight = 67,000) were dissolved in 1 ml of 50 mM Na₂CO₃, pH 9.5. In a separate vessel, 10 mg of Biotin-amidocaproate N-hydroxysuccinimide ester (Sigma, Molecular weight = 454) were dissolved in 200 ul dry N,N- dimethylformamide (DMF).

A 50-fold molar excess of the active biotin ester was added to the BSA and the reaction allowed to take place for 3 hours at room temperature. Subsequently, the reaction mixture was diluted to 2.5 ml by addition of 10 mM HEPES buffer, pH 7.5, containing 0.9% NaCl and 0.5% sodium azide and passed over a PD-10 column (Pharmacia LKB Biotechnology), equilibrated with the same buffer solution. The process was repeated by rechromatographing the biotinylated BSA on newly prepared PD-10 columns, using half of the original effluent for each run. The purified product was stored at +4 degrees until used.

### 5. Preparation of Streptavidin Microtitration Plates

Nunc high binding plates were coated with 200 ul/well of the biotinylated BSA diluted to 100 ng/ml in 50 mM K₂HPO₄, buffer, pH 9.0, containing 0.9% NaCl and 0.05% sodium azide and incubated overnight at room temperature. The plates were then washed six times (using a plate washer and Wash solution from Delfia, Pharmacia Wallac); a solution (200 ul per well) of streptavidin (BRL, Bethesda, MD), diluted in Assay Buffer (Delfia, Pharmacia Wallac) to 2 ug/ml were added and the plates were incubated at room temperature for 3 hours, and finally washed 6 times before use.

### 6. Preparation of Lymphocytes

### a. Ficoll-Hypaque Gradient Centrifugation

Five ml Ficoll hypaque (Pharmacia LKB Biotechnology) were placed in a 15 ml centrifuge tube and a volume (5ml) of anti-coagulated blood were carefully placed on top of the Ficoll hypaque and centrifuged in a table-top centrifuge at 2,500 rpm for 20 minutes. The plasma was removed and the lymphocyte band was collected with a pasteur pipette. The lymphocytes were added to 10 ml of 1xSSC (0.015 M sodium citrate buffer, pH 7.5, containing 0.15 M sodium chloride) and counted. The cells were then collected by centrifugation at 1500 rpm for 5 minutes and resuspended in a volume of 1xSSC to give a final concentration of 10⁶ cells per ml. Clinical samples were kept frozen at this concentration until subjected to the lysis procedure (Example 7); for quantitative measurements using COS 10-11.1 cells, dilutions of these cells in thawed suspensions of ficoll purified cells from healthy donors were prepared by mixing the cells, centrifuging in an Eppendorf centrifuge and resuspending the resulting pellet to a concentration of one million cells per ml to be used in the lysis procedure (Example 7).

### b. Magnetic Bead Separation

One ml of human blood from an HIV-negative donor was mixed with 1 ml phosphate-buffered saline containing varying numbers (0, 50 and 500) of cells derived from U937 myeloma cells prepared in an analagous manner to the procedure described in to Example 1 and containing one genome of truncated proviral HIV-DNA per cell.

As appropriate control, tubes were prepared which contained all components except the cells derived from U937. After mixing, commercially available Dynabeads, Product no. 11106, containing covalently attached antibody to the human CD4 receptor were added in a 20 fold excess (100 ul suspension) to each tube and incubation was allowed to take place with occasional shaking during 30 minutes. The beads were separated using a magnet and washed three times, and finally suspended in 0.5 ml of 1xSSC for the lysis step (See Example 7)

### 7. Lysis procedure

Aliquots (250 ul) of the cell suspension (250,000 cells total) were placed in a sterile 0.5 ml Eppendorf tube and centrifuged at 4000 rpm for 2 minutes in an Eppendorf centrifuge. After removing the supernatant a mixture of 30 ul of distilled water and 10 ul of a solution containing 5 uM concentration of the relevant primer pair (in 10 mM Tris-HCl buffer, pH 7.7) were added to resuspend the cell pellet.

When analyzing cells for presence of HIV proviral DNA, the following primers were used, capable of hybridizing to the LTR region of HIV and initiating synthesis of a portion of that region: 5' GGAACCCACTGCTTAAGCC 3' (S1) and

5' GGTCTGAGGGATCTCTAG 3' (S2)

The cells were then autoclaved for 10 min (in some studies for 20 minutes) at 121 C with fast exhaust, cooled and subjected to amplification according to Example 8.

### 8. Amplification procedure

The steps involving amplification were carried out in an area separated from the space designated for the subsequent hybridization steps in order to avoid sample cross-contamination.

Prior to the amplification step via PCR, a PCR Master mix was prepared as follows: One ml contains 720 ul 2x Amplification buffer (20 mM Tris-HCl, pH 8.4, containing 100 mM NaCl, 5 mM MgCl₂, 0.4 mg/ml gelatin and 400 uM of each dNTP (Pharmacia LKB Biotechnology) and 140 ul TAQ polymerase (0.1 U/ml, in 10 mM Tris-HCl, pH 7.4, containing 300 mM KCl, 2mM DTT, 100 uM EDTA, 200 ug/ml Gelatin and 50% Glycerol. This mixture was kept on ice until used.

The freshly resuspended lymphocytes were subjected to autoclaving and then cooled on ice as described in Example 7. Thereafter the digest was centrifuged briefly in an Eppendorf centrifuge to pellet condensed water. Seventy ul of the PCR Master Mix were added to each tube, followed by 100 ul of light mineral oil (Sigma M-3516). The tubes were capped mixed well and centrifuged again in an Eppendorf instrument.

The tubes were then transferred to a Thermocycler (Perkin-Elmer Cetus), programmed for the following thermocycle program: 95°C, 50 seconds; 55°C 2 minutes; 73°C 2 minutes. Thirty cycles were performed. Effective heat transfer was achieved by adding one drop of mineral oil to each well.

After cycling, the samples were removed from the Thermocycler and transferred to a separate zone for performance of the ABC assay. Care was taken not to open the tubes until they had been placed in the isolated zone.

### 9. Hybridization and Detection

Prior to hybridization, a solution was mixed as follows:

Per 1 ml of hybridization solution, to 500 ul of 2xhybridization buffer stock ( 110 mM HEPES buffer, containing 1.1 M NaCl. 220 uM EDTA and 0.1% Tween-20), 480 ul distilled H₂O, 10 ul biotinylated BSA (5.6 ug/ml) and 10 ul Europium-labeled oligonucleotide (5.6 ug/ml) were added.

Ten ul of the amplification product were transferred into a 0.5 ml Eppendorf tube. The transferred solution was heated at 95°C for 8 minutes, then cooled on ice for 2 minutes, centrifuged (using the Eppendorf centrifuge). Then, 90 ul of the hybridization solution were added, mixed and centrifuged. The mixture was kept at 55°C for one hour in an incubator. Subsequently, the hybridization reaction mixture (100 ul) was transferred to a well of the streptavidin-coated microtiter plate prepared in Example 5. One hundred ul of a solution (Assay Buffer, Delfia Pharmacia Wallac, supplemented with sodium chloride to yield a final NaCl concentration of 1 mole/liter), were then added to each well, and the plates were incubated with shaking (Plateshake, Pharmacia Wallac) for 1 hour at room temperature. Then, the plates were washed 6 times with the Platewasher using the Wash solution (see above). After removing the last Wash solution, each well received 200 ul Enhancement Solution (Delfia,Pharmacia Wallac, cat. no. 1244-105). The Delfia enhancement solution and its use is described in U.S. Patent No. 4,565,790 issued on January 21, 1986. Briefly, the europium was released and the fluorescence of the label was enhanced by adding 200 ul of a solution containing 0.1 M acetate buffer adjusted to pH 3.2 with potassium hydrogen phthalate containing 15 uM of 2-naphthoyltriflouroacetone (2-NTA), 50 uM trioctylphosphineoxide (TOPO) and O.a percent Triton X-100. The microtiter plate was again shaken for 25 minutes at room temperature and subsequently, the fluorescence was determined using an Arcus TR-fluorometer (Pharmacia Wallac)to measure time resolved flourescence. The fluorescence was measured at various indicated times after addition by means of a single photon counting time-resolved fluorometer using a Xenon flash lamp (1000HZ) for a total measuring time of 1 second; with a 50 microsecond delay time and a 250 microsecond counting time. This time-resolved fluorometer and related instrumentation methods are described in U.S. Patent No. 4,058,732 where a sample is excited by a laser pulse of a short duration, the fluorescence being detected only when the fluorescence from noise sources has declined.

### 10. Dose-Reponse Relationship Following Isolation of Cells and Heat Treatment

### a. Ficoll-hypaque isolated cells

Table 2 shows the the standard curve obtained when combining Ficoll-hypaqe isolation (Example 6A) and heat treatment according to Example 7 by autoclaving for 10 minutes, of a series of COS 10-11.1 cells diluted into a background of 250,000 lymphocytes isolated from a healthy donor. In this case, however, the samples were PCR amplified using primers S1 and S2 (Table 1) and HIV-specific nucleic acid detected using Europiun labeled 59 and bistinylated S11 in the ABC sandwich assay (Example 9)

**TABLE 2**

| Sample | COS 10-11.1 Cells per sample | CPS (Average of 3) |
|---|---|---|
| 1 | 0 | 1302 |
| 2 | 5 | 12441 |
| 3 | 10 | 33102 |
| 4 | 20 | 31663 |
| 5 | 40 | 101276 |
| 6 | 80 | 175246 |
| 7 | 160 | 345752 |
| 8 | 320 | 575714 |
| 9 | 640 | 636176 |

The data in Table 2 demonstrates that the assay system is sensitive enough to detect HIV in as few as 5 cells out of a background of one million, and that the assay is approximately linear in the range of 5 to 40 10-11.1 COS cells per million of background cells. Each data point is the average of three independent assays. Similar results were obtained when the cells were heated at 115C for 30 minutes.

### b. Cells isolated by contacting with magnetic beads containing covalent anti CD4 antibody

Table 3 shows the data obtained by the methods of Example 6a when varying amounts of COS 10-11.1 cells containing proviral DNA were first added to human blood samples and isolated via surface-bound CD4 receptors reacting with anti-CD4 antibody covalently attached to magnetic beads (Example 6B).

**TABLE 3**

| Sample | U937 cells containing Proviral HIV DNA added to blood, then CD4 cells reisolated | CPS after heat treatment, PCR, ABC |
|---|---|---|
| 1 | 0 | 3484 |
| 2 | 50 | 36332 |
| 3 | 500 | 77571 |

### 11. Clinical samples.

To further examine the utility of the present invention on actual clinical specimens, 8 lymphocyte samples each containing about 10⁶ cells were obtained from HIV-1 infected individuals. These samples had been frozen in media containing 40% fetal bovine serum and 10% DMSO, and were divided into aliquots of 250 000 cells, pelleted by centrifugation (3000 rpm, 5 minutes) re-suspended in 30 ul of H₂O prior to the PCR. Lymphocyte samples (250,000 cells per test) from HIV-1 infected individuals were tested as duplicates in the PCR based ABC-hybridization assay according to Example 10. Cells from 3 healthy seronegative individuals were used as negative controls. Twice mean signal from the average of the 3 negatives (5757 cps) multiplied was considered to be the cut-off for a positive sample in the assay. As shown in Table 4, the samples from HIV-1 infected individuals all gave strongly positive signals in the assay, with the lowest positive signal over 6-fold higher than the negative controls.

**TABLE 4**

| Detection of HIV-1 Nucleic Acid from Clinical Samples | | | | |
|---|---|---|---|---|
| Patient # | Infect/Uninfect. | Signals (cps) | Mean signal (cps) | +/- |
| 1 | Uninfect. | 5761 | | |
| | | 6495 | 6128 | - |
| 2 | " | 7759 | | |
| | | | | |
| | | 4198 | 5978 | - |
| 3 | " | 5644 | | |
| | | 4687 | 5165 | - |
| 4 | Infect. | 81520 | | |
| | | 78366 | 80039 | + |
| 5 | " | 99960 | | |
| | | 106365 | 103163 | + |
| 6 | " | 81520 | | |
| | | 72400 | 76960 | + |
| 7 | " | 93390 | | |
| | | 166483 | 129937 | + |
| 8 | " | 57203 | | |
| | | 94610 | 75906 | + |
| 9 | " | 47054 | | |
| | | 29227 | 38140 | + |
| 10 | " | 204141 | | |
| | | 210630 | 207385 | + |
| 11 | " | 169208 | | |
| | | 170787 | 169997 | + |

Similarly, a separate set of clinical samples taken from HIV positive donors were tested following ficoll-hypaque centrifugation to isolate the cells and autoclaving for 10 minutes as pretreatment according to the method of the invention. Table 5 shows the results of the assay, demonstrating all samples scored positive (the negative control in this assay was 5841 cps).

**TABLE 5**

| Detection of HIV-1 Nucleic Acid from Clinical Samples following heat treatment by autoclaving | | | | |
|---|---|---|---|---|
| Negative Control: 5841 cps | | | | |
| Patient # | Infect/Uninfect. | Signals (cps) | Mean signal (cps) | +/- |
| 1 | Infected | 111047 | | |
| | | 164694 | 137871 | + |
| 2 | " | 150520 | | |
| | | 196732 | 173,626 | + |
| 3 | " | 274978 | | |
| | | 177572 | 226275 | + |
| 4 | " | 330301 | | |
| | | 369231 | 349766 | + |
| 5 | " | 192594 | | |
| | | 55667 | 97256 | + |
| 6 | " | 105843 | | |
| | | 88670 | 97256 | + |
| 7 | " | 213411 | | |
| | | 218090 | 215750 | + |
| 8 | " | 225451 | | |
| | | 292718 | 259084 | + |
| 9 | " | 119131 | | |
| | | 127404 | 123267 | + |
| 10 | " | 119311 | | |
| | | 110950 | 115131 | + |
| 11 | " | 277111 | | |
| | | 292253 | 284682 | + |
| 12 | " | 130688 | | |
| | | 194879 | 162784 | + |
| 13 | " | 289051 | | |
| | | 238579 | 263815 | + |
| 14 | " | 216327 | | |
| | | 256117 | 236222 | + |
| 15 | " | 124181 | | |
| | | 116829 | 120505 | + |
| 16 | " | 79010 | | |
| | | 99764 | 89387 | + |
| 17 | " | 86005 | | |
| | | 70350 | 78177 | + |

### 12. Detection of Other Retroviruses

### a. Detection of HTLV II

HTLV-II virus was specifically detected in cells (ATCC #CRL8066) using oligonucleotides P1 and P2 of Table 1 as 5' and 3' as PCR primers, biotinylated probe P3 and Europium labeled P4 (Table 1) using the heat pretreatment method of Example 7. Briefly, the cells (250,000 cells in H₂O) were heat treated and centrifuged prior to placing them in a solution containing 10 mM Tris-HCl at pH 8.4, 2.5 mM MgCl₂, 200 mM dNTP's, 0.2 mg/ml gelatin, 50 mM NaCl, and 0.5 uM of each of the PCR primers P1 and P2 (Table 1) and 1U of TAQ Polymerase (Perkin-Elmer Cetus, Norwalk, CT) in a 100 ul total volume. The tubes containing the reaction mix were incubated in a Thermal Cycler (Perkin-Elmer Cetus) using the following program: 95C for 50 seconds, 63 for 2 minutes, and 73C for 2 minutes for a total of 27 cycles.

Ten percent of the total PCR mix produced above was assayed in the ABC sandwich hybridization (Example 9), except that the biotinylated probe P3 and the Europium labeled probe was P4 (Table 1) were used to detect HTLV-II virus.

The heat pretreatment in combination with the ABC hybridization assay was permitted detection of very low numbers of infected cells in a sample containing 200,000 normal lymphocytes as shown in Table 6A.

**TABLE 6A**

| HTLV-II Detection Assay | |
|---|---|
| Infected Cells¹ | CPS² |
| 3 | 1,072 |
| 6 | 2,058 |
| 12 | 6,884 |
| 24 | 11,121 |
| 48 | 25,715 |
| 72 | 38,176 |
| 96 | 48,855 |
| 144 | 78,387 |
| 192 | 100,807 |

| | |
|---|---|
| 1) The indicated number of infected cells were mixed with 200,000 normal lymphocytes. | |
| 2) 200,000 normal lymphocytes gave a value of 377 counts per minute as measured by time-resolved fluorometry as described. Indicated CPS's are an average of 3 samples after subtracting the background. | |

### b. Detection of HTLV-I

HTLV-I virus was specifically detected using oligonucleotides P5 and P6 of Table 1 as 5' and 3' PCR primers, biotinylated probe P7 and Europium labeled P8 (Table 1).

Briefly, the cells (250,000 cells in H₂O) were heat treated and centrifuged prior to placing the cell sample in a solution containing 10 mM Tris-HCl at pH 8.4, 2.5 mM MgCl₂, 200 uM dNTP's, 0.2 mg/ml gelatin, 50 mM Nacl, and 0.5 uM of each of the PCR primers P5 and P6 (Table 1) and 1U of TAQ Polymerase (Perkin-Elmer Cetus, Norwalk, CT) in a 100 ul total volume. The tubes containing the reaction mix where incubated in a Thermal Cycler (Perkin-Elmer Cetus) using the following program: 95C for 50 seconds, 60C for 2 minutes, and 73C for 2 minutes for a total of 30 cycles.

Ten percent of the total PCR mix produced above was assayed in the ABC sandwich hybridization (Example 9), except that the biotinylated probe P7 and the Europium labeled probe was P8 (Table 1) were used to detect HTLV-I virus.

The heat pretreatment in combination with the ABC hybridization assay permitted detection of very low numbers of infected cells in a sample of lymphocytes as shown in Table 6B.

**TABLE 6B**

| HTLV-I Clinical Samples | |
|---|---|
| Infected Cells | CPS¹ |
| Sample 1A | 11,506 |
| Sample 2A | 17,507 |
| Sample 3A | 35,123 |
| Sample 4A | 288,950 |
| Sample 5A (uninfected) | 1,240 |
| Salmon Sperm DNA | 946 |
| 10 HTLV-II infected cells | 1,175 |
| 10-100 cells of HVT102 (ATCC #TIB162) | 60,000 |

| | |
|---|---|
| 1) The number of counts per minute as measured by time-resolved fluorometry as described. | |

### c. Detection of HIV-2

HIV-2 virus was specifically detected in DNA isolated from HIV-2 infected MoLT-4 cells (ATCC# CRL1582) using oligonucleotides P9 and P10 of Table 1 as 5' and 3' PCR primers, biotinylated probe Pll and Europium labeled P12 (Table 1). Briefly, DNA was isolated from the cells and diluted to the indicated cell equivalent amount using the heat pretreatment method of Example 7. The DNA was admixed with a solution containing 10 mM Tris-HCl at pH 8.4, 2.5 mM MgCl₂, 200 uM dNTP's, 0.2 mg/ml gelatin, 50 mM NaCl, and 0.5 uM of each of the PCR primers P1 and P2 (Table 1) and 1U of TAQ Polymerase (Perkin-Elmer Cetus, Norwalk, CT) in a 100 ul total volume. The tubes containing the reaction mix where incubated in a Thermal Cycler (Perkin-Elmer Cetus) using the following program: 95C for 50 seconds, 65C for 2 minutes, and 73C for 2 minutes for a total of 30 cycles.

Ten percent of the total PCR mix produced above was assayed in the ABC sandwich hybridization according to Example 9, except that the biotinylated probe P11 and the Europium labeled probe was P12 (Table 1) were used to detect HIV-2 virus.

The heat pretreatment in combination with the ABC hybridization assay was able to detect very low amounts of DNA as shown in Table 6C. Prior to assay the DNA isolated from the HIV-2 infected MoLT-4 cells was digested with the restriction endonuclease Eco RI according to the enzyme manufacturers instructions.

**TABLE 6C**

| HIV-2 ABC Assay | |
|---|---|
| Cell Equivalent⁽¹⁾ | CPS⁽²⁾ |
| 0 | 784 |
| 1 | 10,425 |
| 5 | 38,969 |
| 10 | 53,413 |
| 25 | 94,093 |
| 50 | 164,810 |
| 100 | 243,532 |
| 250 | 302,904 |
| 500 | 226,220 |

| | |
|---|---|
| (1) Cell equivalents of DNA isolated from HIV-2 infected MoLT-4 (ATCC# CRL1582) cells using phenol extraction as described in Maniatis, et al., Molecular Cloning: A Laboratory Manual, Cold Spring Harbor, NY (1982). | |
| (2) Counts per minute as measured by time-resolved fluorometry as described. | |

### d. Detection of HIV-I

HIV-I virus was specifically detected in COS10-11 cells using PCR probes P13 and P14 of Table 1 as 5' and 3' primers, biotinylated probe P15 and P17, and Europium labeled P16 and P18 (Table 1). Briefly, DNA was isolated from the cells and diluted to the indicated cell equivalent amount using the heat pretreatment method of Example 7. The DNA was admixed with a solution containing 10 mM Tris-HCl at pH 8.4, 2.5 mM MgCl₂, 200 uM dNTP's, 0.2 mg/ml gelatin, 50 mM NaCl, and 0.5 uM of each of the PCR primers P13 and P14 (Table 1) and 1U of TAQ Polymerase (Perkin-Elmer Cetus, Norwalk, CT) in a 100 ul total volume. The tubes containing the reaction mix where incubated in a Thermal Cycler (Perkin-Elmer Cetus) using the following program: 95C for 50 seconds, 65C for 2 minutes, and 73C for 2 minutes for a total of 30 cycles.

Ten percent of the total PCR mix produced above was assayed in the ABC sandwich hybridization according to Example 9, except that both biotinylated probes P16 and P17 were used to detect the + and - PCR amplified strands. The Europium labeled probes P16 and P18 (Table 1) were used to detect HIV-I virus.

The heat pretreatment in combination with the ABC hybridization assay was able to detect very low numbers of cells among 250,000 uninfected lymphocytes as background in each sample as shown in Table 6D.

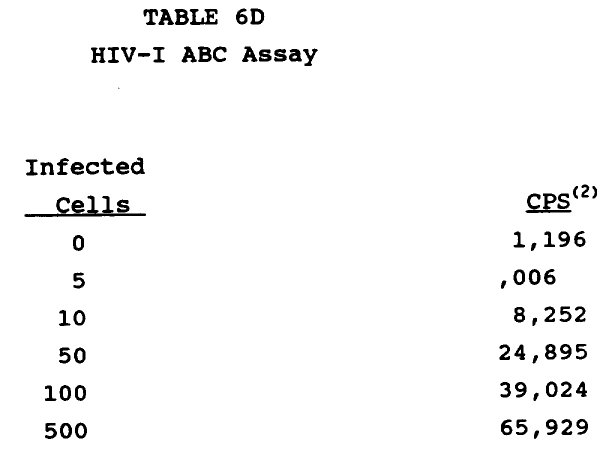

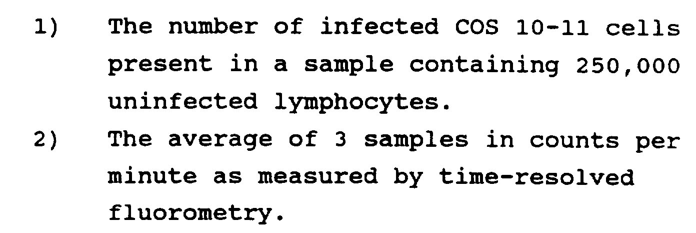

### 13. Effects of Temperature of Cell Lysis

To further examine the effects of temperature pretreatment on the ability to detect a specific genomic DNA sequence, pretreatment of isolated cells at 115C for 10 minutes was compared to pretreatment at 100C for 10 minutes using the methods described in Example 10. The results of this study, whose data are typically shown on Table 7, indicate that pretreatment at 100C did not result in the production of a signal above the average background, whereas pretreatment at 115C did. Thus, pretreatment of the cells at 115C significantly increases the sensitivity of the assay, thereby substantially lowering the amount of a specific genomic DNA sequence that can be detected in a sample.

**TABLE 7**

| Effect of Pretreatment Temperature¹ | | |
|---|---|---|
| Sample No. | 100 Degrees C | 115 Degrees C |
| 1 | 6562² | 39265 |
| 2 | 6984 | 17392 |
| 3 | 3711 | 37823 |
| 4 | 5052 | 22089 |
| 5 | 6720 | 25488 |
| 6 | 7366 | 24356 |
| 7 | 3416 | 15054 |
| 8 | 4520 | 18371 |
| 9 | 3628 | 5453 |
| 10 | 11130 | 20305 |
| Average | 5909 | 22560 |
| Average Background | 8115 | 7824 |

| | | |
|---|---|---|
| 1. HIV-positive control cells (COS 10-11.1 cells) diluted into a background of HIV-negative lymphocytes at a ratio of 1:1x10⁵. | | |
| 2. Counts per minute (CPM) as determined by time-resolved fluorometry as described. | | |

### 14. Serum Protein Prevents Detection By PCR Assay

The effect of serum on the ability to detect a specific nucleic acid sequence in cells was examined. Cell samples substantially free of extracellular (serum) protein were prepared to contain 50 COS 10-11.1 cells admixed in a background of 250,000 normal human lymphocytes. Different amounts of serum (10 ul or 35 ul) were substituted for water in some samples to produce PCR reaction admixtures containing about 29% (v/v) and 100% (v/v) extracellular serum protein, respectively, as shown in Table 8. The samples were then pretreated by heating to 115C for 30 minutes, after which buffer, dNTP, primers and polymerase were added as described in Example 10. An oil overlay was applied and 30 cycles of the PCR were run. The results of this study, shown in Table 8, indicate that the presence of serum protein prevents achieving even negative control (background) values in the PCR assay. Similar results are obtained using plasma instead of serum.

**TABLE 8**

| HIV-Positive Cells | | | | |
|---|---|---|---|---|
| 29% v/v #1 | Serum #2 | 100% v/v #1 | Serum #2 | Substantially Free |
| 667 | 744 | 337 | 4480 | 47,151 |
| 438 | 503 | 419 | 633 | 29,285 |
| 491 | 603 | 486 | 553 | 33,074 |
| 532 | 617 | 414 | 1889 | 36,503 |
| | | | | |

| HIV-Negative Cells | | | | |
|---|---|---|---|---|
| 29% v/v Serum | | | Substantially Free | |
| 4024 | | | 9366 | |
| 2122 | | | 5035 | |
| 526 | | | 3948 | |
| 2226 | | | 6117 | |
| 1. Counts per minute as determined by time resolved fluorometry as described in Example 9. | | | | |

## Claims

1. A method for releasing genomic DNA from cells, which method comprises using antibodies attached to a solid support which bind to said genomic DNA-containing cells to isolate the cells in an aqueous medium containing less than 80 mg/ml extracellular protein, and subjecting said cells to a temperature of at least 105 degrees C for a time period of at least 5 minutes.

2. The method of claim 1, wherein the solid support is paramagnetic.

3. The method of claim 1 or 2, wherein the solid support is in the form of a bead.

4. The method of any one of claims 1 to 3, wherein said antibodies specifically react with a cell surface receptor that is the CD4 cell surface receptor, the CD8 cell surface receptor, the CD2 cell surface receptor or the CD3 cell surface receptor.

5. The method of any one of claims 1 to 4, wherein said time period is in the range of 5 to 20 minutes.

6. The method of any of claims 1 to 5, wherein the heat treatment of the isolated cells is carried out at a temperature in the range of 105 degrees C to 125 degrees C for a time period of at least about 10 minutes.

7. The method of claim 6, wherein said temperature is in the range of 110 to 125 degrees C and said time period is in the range of 15-30 minutes.

8. The method of any one of claims 1 to 7, wherein said aqueous medium contains less than 40 mg/ml extracellular protein.

9. The method of any one of claims 1 to 8, wherein said aqueous medium is substantially free of hemoglobin and red blood cells.

10. The method of any one of claims 1 to 9, wherein the heat treatment of the isolated cells is carried out at superatmospheric steam pressure.

11. The method of any one of claims 1 to 10, wherein said genomic DNA containing cells are isolated from a blood sample.

12. The method of any one of claims 1 to 11, wherein a portion of said released genomic DNA is subjected to at least one cycle of a polymerase chain reaction using a first polynucleotide synthesis primer capable of initiating synthesis of a complement of a specific nucleic acid sequence of said genomic DNA and a second polynucleotide synthesis primer capable of initiating synthesis of said specific nucleic acid sequence.

13. The method of claim 12, wherein, under hybridizing conditions, the polymerase chain reaction product is treated with an affinity-labeled polynucleotide probe and a reporter group-labeled polynucleotide probe, each of said probes being capable of hybridizing to different regions of a single-stranded DNA of said polymerase chain reaction product to form a DNA duplex having affinity- and reporter group-labels linked thereto, and treating said DNA duplex, under binding conditions, with a solid-phase affinity sorbent capable of binding said affinity-label of said DNA duplex to form a solid-phase reporter group-labeled DNA duplex, and determining the amount of said solid-phase reporter group-labeled DNA duplex formed.

14. The method of claim 13, wherein said affinity label is biotin to be bound to solid phase avidin or streptavidin, or is dinitrophenylated nucleic acid to be bound to solid phase anti-dinitrophenyl antibody.

15. The method of claim 13 or 14, wherein said reporter group is an enzyme, a radioactive isotope, a fluorescent label, or an acridinium ester.

16. The method of any of claims 12 to 15, wherein the specific nucleic acid sequence is a pathogen-specific nucleic acid sequence.

17. The method of claim 16, wherein said pathogen-specific nucleic acid sequence is a sequence corresponding to a human retroviral pathogen.

18. The method of any of claims 13 to 17, wherein the primers and probes are specific for a blood borne pathogen, the human immunodeficiency virus, the human lymphotropic virus type I or the human lymphotropic virus type II.

19. The method of claim 18, wherein the primers and probes are specific for
a) human lymphotropic virus Type I and said first polynucleotide primer has a nucleotide sequence corresponding to the formula, TGCAGCTGGCCCATATCCTGC, and said second polynucleotide primer has a nucleotide sequence corresponding to the formula, TAGGGAAGCCATTGTGGCCTC,
b) human lymphotropic virus type II and said polynucleotide primer has a nucleotide sequence corresponding to the formula, CAACAATTAGCAGCCGTCCTC, and said second polynucleotide primer has a nucleotide sequence corresponding to the formula, AGTGCCTGGAGGGTTAGCTG,
c) HIV-2, and said first polynucleotide primer has a nucleotide sequence corresponding to the formula, CCACGCTTGCTTGCTTAAAGACCTC, and said second polynucleotide primer has a nucleotide sequence corresponding to the formula, ATTTTCCTGCCTCGGTTTCCCAAAG, or
d) HIV-1, and
(i) said first polynucleotide primer has a nucleotide sequence corresponding to the formula, CTTAAGACAGCAGTACAAATG, and said second polynucleotide primer has a nucleotide sequence corresponding to the formula, TGTCCCTGTAATAAACCCG or
(ii) said first polynucleotide primer has a nucleotide sequence corresponding to the formula, GGAACCCACTGCTTAAGCC, and said second polynucleotide primer has a nucleotide sequence corresponding to the formula,

GGTCTGAGGGATCTCTAG.

20. The method of any of claims 12 to 19, wherein said aqueous medium has a pH value in the range of 6.0 to 8.5.

21. Use of the method of any of claims 1 to 20 for detecting a specific nucleic acid contained in a blood sample.

## Patentansprüche

1. Verfahren zur Freisetzung genomischer DNA aus Zellen, wobei man an einen festen Träger gebundene Antikörper verwendet, die an die die genomische DNA enthaltenden Zellen binden, um die Zellen in einem wäßrigen Medium, das weniger als 80 mg/ml extrazelluläres Protein enthält, zu isolieren, und man die Zellen einer Temperatur von mindestens 105°C für eine Zeitspanne von mindestens 5 Minuten unterwirft.

2. Verfahren nach Anspruch 1, wobei der feste Träger para-magnetisch ist.

3. Verfahren nach Anspruch 1 oder 2, wobei der feste Träger in Form eines Kügelchens vorliegt.

4. Verfahren nach einem der Ansprüche 1 bis 3, wobei die Antikörper spezifisch mit einem Zelloberflächen-Rezeptor, der der CD4-Zelloberflächen-Rezeptor, der CD8-Zelloberflächen-Rezeptor, der CD2-Zelloberflächen-Rezeptor oder der CD3-Zelloberflächen-Rezeptor ist, reagieren.

5. Verfahren nach einem der Ansprüche 1 bis 4, wobei die Zeitspanne im Bereich von 5 bis 20 Minuten liegt.

6. Verfahren nach einem der Ansprüche 1 bis 5, wobei man die Wärmebehandlung der isolierten Zellen bei einer Temperatur im Bereich von 105°C bis 125°C über eine Zeitspanne von mindestens etwa 10 Minuten durchführt.

7. Verfahren nach Anspruch 6, wobei die Temperatur im Bereich von 110 bis 125°C liegt und die Zeitspanne im Bereich von 15 bis 30 Minuten liegt.

8. Verfahren nach einem der Ansprüche 1 bis 7, wobei das wäßrige Medium weniger als 40 mg/ml extrazelluläres Protein enthält.

9. Verfahren nach einem der Ansprüche 1 bis 8, wobei das wäßrige Medium im wesentlichen frei von Hämoglobin und Erythrozyten ist.

10. Verfahren nach einem der Ansprüche 1 bis 9, wobei man die Wärmebehandlung der isolierten Zellen bei einem superatmosphärischen Dampfdruck durchführt.

11. Verfahren nach einem der Ansprüche 1 bis 10, wobei man die genomische DNA enthaltenden Zellen aus einer Blutprobe isoliert.

12. Verfahren nach einem der Ansprüche 1 bis 11, wobei man einen Teil der freigesetzten genomischen DNA mindestens einem Zyklus einer Polymerase-Ketten-Reaktion unter Verwendung eines ersten Polynukleotidsynthese-Primers, der die Synthese eines Komplements einer spezifischen Nukleinsäuresequenz der genomischen DNA initiieren kann, und eines zweiten Polynukleotidsynthese-Primers, der die Synthese der spezifischen Nukleinsäuresequenz initiieren kann, unterwirft.

13. Verfahren nach Anspruch 12, wobei man unter hybridisierenden Bedingungen das Polymerase-Ketten-Reaktionsprodukt mit einer affinitätsmarkierten Polynukleotidsonde und einer Reportergruppe-markierten Polynukleotidsonde behandelt, wobei jede der Sonden mit unterschiedlichen Regionen einer einzelsträngigen DNA des Polymerase-Ketten-Reaktionsprodukts unter Bildung einer doppelsträngigen DNA, an die die Affinitäts- und Reportergruppen-Marker gebunden sind, hybridisieren kann, und die doppelsträngige DNA unter bindenden Bedingungen mit einem Festphasen-Affinitiäts-Absorptionsmittel behandelt, das den Affinitätsmarker der doppelsträngigen DNA binden kann, wobei ein Doppel strang aus einer mit einer Festphasen-Reportergruppe markierten DNA gebildet wird, und die Menge des gebildeten, mit einer Festphasen-Reportergruppe markierten DNA-Doppelstrangs bestimmt.

14. Verfahren nach Anspruch 13, wobei der Affinitätsmarker Biotin zur Bindung an Festphasen-Avidin oder -Streptavidin ist oder eine dinitrophenylierte Nukleinsäure zur Bindung an einen Festphasen-Antidinitrophenyl-Antikörper ist.

15. Verfahren nach Anspruch 13 oder 14, wobei die Reportergruppe ein Enzym, ein radioaktives Isotop, ein fluoreszierender Marker oder ein Acridiniumester ist.

16. Verfahren nach einem der Ansprüche 12 bis 15, wobei die spezifische Nukleinsäuresequenz eine Krankheitserreger-spezifische Nukleinsäuresequenz ist.

17. Verfahren nach Anspruch 16, wobei die Krankheitserregerspezifische Nukleinsäuresequenz eine einem menschlichen retroviralen Krankheitserreger entsprechende Sequenz ist.

18. Verfahren nach einem der Ansprüche 13 bis 17, wobei die Primer und Sonden für einen über den Blutweg verbreiteten Krankheitserreger, das menschliche Immunschwäche-Virus, das menschliche lymphotrope Virus Typ I oder das menschliche lymphotrope Virus Typ II spezifisch sind.

19. Verfahren nach Anspruch 18, wobei die Primer und Sonden spezifisch sind für
a) das menschliche lymphotrope Virus Typ I, und der erste Polynukleotid-Primer besitzt eine Nukleotidsequenz, entsprechend der Formel TGCAGCTGGCCCATATCCTGC, und der zweite Polynukleotid-Primer besitzt eine Nukleotidsequenz, entsprechend der Formel TAGGGAAGCCATTGTGGCCTC,
b) das menschliche lymphotrope Virus Typ II, und der Polynukleotid-Primer besitzt eine Nukleotidsequenz, entsprechend der Formel CAACAATTAGCAGCCGTCCTC, und der zweite Polynukleotid-Primer besitzt eine Nukleotidsequenz, entsprechend der Formel AGTGCCTGGAGGGTTAGCTG,
c) HIV-2, und der erste Polynukleotid-Primer besitzt eine Nukleotidsequenz, entsprechend der Formel CCACGCTTGCTTGCTTAAAGACCTC, und der zweite Polynukleotid-Primer besitzt eine Nukleotidsequenz, entsprechend der Formel ATTTTCCTGCCTCGGTTTCCCAAAG, oder
d) HIV-1, und
(i) der erste Polynukleotidprimer besitzt eine Nukleotidsequenz, entsprechend der Formel CTTAAGACAGCAGTACAAATG, und der zweite Polynukleotid-Primer besitzt eine Nukleotidsequenz, entsprechend der Formel TGTCCCTGTAATAAACCCG, oder
(ii) der erste Polynukleotid-Primer besitzt eine Nukleotidsequenz, entsprechend der Formel GGAACCCACTGCTTAAGCC, und der zweite Polynukleotidprimer besitzt eine Nukleotidsequenz, ensprechend der Formel

GGTCTGAGGGATCTCTAG.

20. Verfahren nach einem der Ansprüche 12 bis 19, wobei das wäßrige Medium einen pH-Wert im Bereich von 6,0 bis 8,5 besitzt.

21. Verwendung des Verfahrens nach einem der Ansprüche 1 bis 20 zum Nachweis einer in einer Blutprobe enthaltenen spezifischen Nukleinsäure.

## Revendications

1. Procédé pour libérer l'ADN génomique de cellules, procédé qui comprend l'utilisation d'anticorps fixés à un support solide qui se lient auxdites cellules contenant de l'ADN génomique pour isoler les cellules dans un milieu aqueux contenant moins de 80 mg/ml de protéines extracellulaires, et l'étape consistant à soumettre lesdites cellules à une température d'au moins 105 degrés C pendant un temps d'au moins 5 minutes.

2. Procédé suivant la revendication 1, dans lequel le support solide est paramagnétique.

3. Procédé suivant la revendication 1 ou 2, dans lequel le support solide est sous forme d'une bille.

4. Procédé suivant l'une quelconque des revendications 1 à 3, dans lequel les anticorps réagissent spécifiquement avec un récepteur de surface cellulaire qui est le récepteur de surface cellulaire CD4, le récepteur de surface cellulaire CD8, le récepteur de surface cellulaire CD2 ou le récepteur de surface cellulaire CD3.

5. Procédé suivant l'une quelconque des revendications 1 à 4, dans lequel le temps est compris dans l'intervalle de 5 à 20 minutes.

6. Procédé suivant l'une quelconque des revendications 1 à 5, dans lequel le traitement thermique des cellules isolées est effectué à une température comprise dans l'intervalle de 105 degrés C à 125 degrés C pendant un temps d'au moins environ 10 minutes.

7. Procédé suivant la revendication 6, dans lequel la température est comprise dans l'intervalle de 110 à 125 degrés C et le temps est compris dans l'intervalle de 15 à 30 minutes.

8. Procédé suivant l'une quelconque des revendications 1 à 7, dans lequel le milieu aqueux contient moins de 40 mg/ml de protéines extracellulaires.

9. Procédé suivant l'une quelconque des revendications 1 à 8, dans lequel le milieu aqueux est pratiquement dépourvu d'hémoglobine et de globules rouges.

10. Procédé suivant l'une quelconque des revendications 1 à 9, dans lequel le traitement thermique des cellules isolées est effectué à une pression de vapeur d'eau supérieure à la pression atmosphérique.

11. Procédé suivant l'une quelconque des revendications 1 à 10, dans lequel les cellules contenant de l'ADN génomique sont isolées d'un échantillon de sang.

12. Procédé suivant l'une quelconque des revendications 1 à 11, dans lequel une portion de l'ADN génomique libéré est soumise à au moins un cycle d'une réaction en chaîne avec une polymérase en utilisant une première amorce de synthèse polynucléotidique capable de déclencher la synthèse d'un complément d'une séquence d'acide nucléique spécifique dudit ADN génomique et une seconde amorce de synthèse polynucléotidique capable de déclencher la synthèse de ladite séquence d'acide nucléique spécifique.

13. Procédé suivant la revendication 12, dans lequel, dans les conditions d'hybridation, le produit de la réaction en chaîne avec une polymérase est traité avec une sonde polynucléotidique marquée par affinité et une sonde polynucléotidique marquée avec un groupe rapporteur, chacune desdites sondes étant capable de s'hybrider à des régions différentes d'un ADN monocaténaire dudit produit de réaction en chaîne avec une polymérase pour former un duplex d'ADN auquel sont liés des marqueurs par affinité et des marqueurs par groupe rapporteur, et ledit duplex d'ADN est traité, dans des conditions de liaison, avec un sorbant d'affinité en phase solide capable de fixer ledit marqueur d'affinité dudit duplex d'ADN pour former un duplex d'ADN marqué avec un groupe rapporteur en phase solide, et la quantité dudit duplex d'ADN marqué avec un groupe rapporteur en phase solide formé est déterminée.

14. Procédé suivant la revendication 13, dans lequel le marqueur d'affinité est la biotine destinée à être liée à de l'avidine ou streptavidine en phase solide, ou consiste en un acide nucléique dinitrophénylé destiné à être lié à un anticorps antidinitrophényle en phase solide.

15. Procédé suivant la revendication 13 ou 14, dans lequel le groupe rapporteur est un enzyme, un isotope radioactif, un marqueur fluorescent ou un ester d'acridinium.

16. Procédé suivant l'une quelconque des revendications 12 à 15, dans lequel la séquence d'acide nucléique spécifique est une séquence d'acide nucléique spécifique d'un agent pathogène.

17. Procédé suivant la revendication 16, dans lequel la séquence d'acide nucléique spécifique d'un agent pathogène est une séquence correspondant à un agent pathogène rétroviral humain.

18. Procédé suivant l'une quelconque des revendications 13 à 17, dans lequel les amorces et les sondes sont spécifiques d'un agent pathogène issu du sang, du virus d'immunodéficience humaine, du virus lymphotrope humain de type I ou du virus lymphotrope humain du type II.

19. Procédé suivant la revendication 18, dans lequel les amorces et les sondes sont spécifiques
a) du virus lymphotrope humain de Type I, et la première amorce polynucléotidique a une séquence de nucléotides répondant à la formule TGCAGCTGGCCCATAITCCTGC, et ladite seconde amorce polynucléotidique a une séquence de nucléotides répondant à la formule, TAGGGAAGCCATTGTGGCCTC,
b) du virus lymphotrope humain de type II, et l'amorce polynucléotidique a une séquence de nucléotides répondant à la formule CAACAATTAGCAGCCGTCCTC, et la seconde amorce polynucléotidique a une séquence de nucléotides répondant à la formule, AGTGCCTGGAGGGTTAGCTG,
c) du HIV-2, et la première amorce polynucléotidique a une séquence de nucléotides répondant à la formule, CCACGCTTGCTTGCTTAAAGACCTC, et la seconde amorce polynucléotidique a une séquence de nucléotides répondant à la formule, ATTTTCCTGCCTCGGTTTCCCAAAG, ou
d) du HIV-1, et
(i) la première amorce polynucléotidique a une séquence de nucléotides répondant à la formule, CTTAAGACAGCAGTACAAATG, et la seconde amorce polynucléotidique a une séquence de nucléotides répondant à la formule, TGTCCCTGTAATAAACCCG, ou
(ii) la première amorce polynucléotidique a une séquence de nucléotides répondant à la formule, GGAACCCACTGCTTAAGCC, et la seconde amorce polynucléotidique a une séquence de nucléotides répondant à la formule,

GGTCTGAGGGATCTCTAG.

20. Procédé suivant l'une quelconque des revendications 12 à 19, dans lequel le milieu aqueux a une valeur de pH comprise dans l'intervalle de 6,0 à 8,5.

21. Utilisation du procédé suivant l'une quelconque des revendications 1 à 20 pour la détection d'un acide nucléique spécifique présent dans un échantillon de sang.
